# EUROPEAN PATENT APPLICATION

(11) **EP 3 112 375 A1**
(43) Date of publication of application: **04.01.2017**
(21) Application number: 15175204.5
(22) Date of filing: 03.07.2015
(51) Int. Cl.: C07K 7/06, C07K 14/74, C07K 14/47, G01N 33/50, G01N 33/564, G01N 33/569

(54) **ANTIGENIC PEPTIDES FOR THE DIAGNOSIS, THERAPY MONITORING AND/OR TREATMENT OF PSORIASIS VULGARIS**

(71) Applicant: Ludwig-Maximilians-Universität München, 80539 München (DE)
(72) Inventor: Prinz, Jörg Christoph, 82319 Starnberg (DE); Dornmair, Klaus, 82140 Olching (DE)
(74) Representative: Engelhard, Markus

(57) **Abstract**

The present invention relates to peptides with a conserved amino acid motif and pharmaceutical compositions comprising these peptides. The present invention further relates to the use of the peptides as biomarker, the medical use of the peptides, in particular in the diagnosis, prevention, monitoring and/or treatment of Psoriasis. The present invention relates to complexes of the peptides of the invention with HLA-C monomers or multimers and their use as biomarker and their medical uses, in particular in the treatment of Psoriasis and in monitoring such treatment. The present invention furthermore relates to means and methods for the prevention and/or treatment of Psoriasis, that comprise inhibiting or blocking the interaction of TCR and HLA-C.

SEQUENCE LISTING

<110>  Ludwig-Maximilians-Universität München

<120>  Antigenic peptides for the diagnosis, therapy monitoring and/or
       treatment of Psoriasis vulgaris

<130>  U30631EP

<160>  31

<170>  PatentIn version 3.5

<210>  1
<211>  9
<212>  PRT
<213>  Artificial Sequence

<220>
<223>  Antigenic peptide

<400>  1

Phe Arg Ser Tyr Arg Thr Arg Arg Leu
1               5


<210>  2
<211>  9
<212>  PRT
<213>  Artificial Sequence

<220>
<223>  Antigenic peptide

<400>  2

Phe Arg His Arg Arg Thr Leu Arg Leu
1               5


<210>  3
<211>  9
<212>  PRT
<213>  Artificial Sequence

<220>
<223>  Antigenic peptide

<400>  3

Phe Arg Cys Arg Gln Tyr Arg Arg Leu
1               5


<210>  4
<211>  9
<212>  PRT
<213>  Artificial Sequence

<220>
<223>  Antigenic peptide

<400>  4

Phe Arg Trp Tyr Arg Val Arg Arg Leu
1               5


<210>  5
<211>  9
<212>  PRT
<213>  Artificial Sequence

<220>
<223>  Antigenic peptide

<400>  5

Arg Arg Asn Trp Asn Tyr Arg Arg Leu
1               5


<210>  6
<211>  9
<212>  PRT
<213>  Artificial Sequence

<220>
<223>  Antigenic peptide

<400>  6

Gly Arg Ala Ser Arg Thr Arg Arg Leu
1               5


<210>  7
<211>  9
<212>  PRT
<213>  Artificial Sequence

<220>
<223>  Antigenic peptide

<400>  7

Gly Arg Asn Ala Arg Val Arg Arg Leu
1               5


<210>  8
<211>  9
<212>  PRT
<213>  Artificial Sequence

<220>
<223>  Antigenic peptide

<400>  8

Met Arg Ser His Arg Tyr Leu Arg Leu
1               5


<210>  9

<211>  9
<212>  PRT
<213>  Artificial Sequence

<220>
<223>  Antigenic peptide

<400>  9

Arg Arg Tyr Phe Arg Cys Leu Arg Met
1               5


<210>  10
<211>  9
<212>  PRT
<213>  Homo sapiens

<400>  10

Ala Arg His Arg Arg Ser Leu Arg Leu
1               5


<210>  11
<211>  9
<212>  PRT
<213>  Homo sapiens

<400>  11

Arg Arg Gln Arg Arg Ser Arg Arg Leu
1               5


<210>  12
<211>  8
<212>  PRT
<213>  Homo sapiens

<400>  12

Arg Gln Arg Arg Ser Arg Arg Leu
1               5


<210>  13
<211>  9
<212>  PRT
<213>  Homo sapiens

<400>  13

Ala Arg Ser Val Arg Thr Arg Arg Leu
1               5


<210>  14
<211>  9
<212>  PRT
<213>  Homo sapiens

<400>  14

Ser Arg Ala Ser Arg Ala Leu Arg Leu
1               5

<210>  15
<211>  9
<212>  PRT
<213>  Homo sapiens

<400>  15

Leu Arg Ala Gly Arg Ser Arg Arg Leu
1               5

<210>  16
<211>  8
<212>  PRT
<213>  Homo sapiens

<400>  16

Arg Ala Gly Arg Ser Arg Arg Leu
1               5

<210>  17
<211>  9
<212>  PRT
<213>  Homo sapiens

<400>  17

Val Arg Ser Arg Arg Cys Leu Arg Leu
1               5

<210>  18
<211>  8
<212>  PRT
<213>  Homo sapiens

<400>  18

Arg Ser Arg Arg Cys Leu Arg Leu
1               5

<210>  19
<211>  9
<212>  PRT
<213>  Homo sapiens

<400>  19

Arg Arg Phe Cys Arg Thr Ser Arg Met
1               5

<210>  20
<211>  9
<212>  PRT

<213>  Homo sapiens

<400>  20

His Arg His Ser Arg Ile Leu Arg Leu
1               5


<210>  21
<211>  9
<212>  PRT
<213>  Homo sapiens

<400>  21

Ser Arg Ala Val Arg Ala Leu Arg Leu
1               5


<210>  22
<211>  2969
<212>  PRT
<213>  Homo sapiens

<400>  22

Met Asp Pro Arg Asn Thr Ala Met Leu Gly Leu Gly Ser Asp Ser Glu
1               5                   10                  15

Gly Phe Ser Arg Lys Ser Pro Ser Ala Ile Ser Thr Gly Thr Leu Val
            20                  25                  30

Ser Lys Arg Glu Val Glu Leu Glu Lys Asn Thr Lys Glu Glu Glu Asp
        35                  40                  45

Leu Arg Lys Arg Asn Arg Glu Arg Asn Ile Glu Ala Gly Lys Asp Asp
    50                  55                  60

Gly Leu Thr Asp Ala Gln Gln Gln Phe Ser Val Lys Glu Thr Asn Phe
65                  70                  75                  80

Ser Glu Gly Asn Leu Lys Leu Lys Ile Gly Leu Gln Ala Lys Arg Thr
                85                  90                  95

Lys Lys Pro Pro Lys Asn Leu Glu Asn Tyr Val Cys Arg Pro Ala Ile
            100                 105                 110

Lys Thr Thr Ile Lys His Pro Arg Lys Ala Leu Lys Ser Gly Lys Met
        115                 120                 125

Thr Asp Glu Lys Asn Glu His Cys Pro Ser Lys Arg Asp Pro Ser Lys
    130                 135                 140

Leu Tyr Lys Lys Ala Asp Asp Val Ala Ala Ile Glu Cys Gln Ser Glu

145                 150                 155                 160

Glu Val Ile Arg Leu His Ser Gln Gly Glu Asn Asn Pro Leu Ser Lys
                165                 170                 175

Lys Leu Ser Pro Val His Ser Glu Met Ala Asp Tyr Ile Asn Ala Thr
            180                 185                 190

Pro Ser Thr Leu Leu Gly Ser Arg Asp Pro Asp Leu Lys Asp Arg Ala
        195                 200                 205

Leu Leu Asn Gly Gly Thr Ser Val Thr Glu Lys Leu Ala Gln Leu Ile
    210                 215                 220

Ala Thr Cys Pro Pro Ser Lys Ser Ser Lys Thr Lys Pro Lys Lys Leu
225                 230                 235                 240

Gly Thr Gly Thr Thr Ala Gly Leu Val Ser Lys Asp Leu Ile Arg Lys
                245                 250                 255

Ala Gly Val Gly Ser Val Ala Gly Ile Ile His Lys Asp Leu Ile Lys
            260                 265                 270

Lys Pro Thr Ile Ser Thr Ala Val Gly Leu Val Thr Lys Asp Pro Gly
        275                 280                 285

Lys Lys Pro Val Phe Asn Ala Ala Val Gly Leu Val Asn Lys Asp Ser
    290                 295                 300

Val Lys Lys Leu Gly Thr Gly Thr Thr Ala Val Phe Ile Asn Lys Asn
305                 310                 315                 320

Leu Gly Lys Lys Pro Gly Thr Ile Thr Thr Val Gly Leu Leu Ser Lys
                325                 330                 335

Asp Ser Gly Lys Lys Leu Gly Ile Gly Ile Val Pro Gly Leu Val His
            340                 345                 350

Lys Glu Ser Gly Lys Lys Leu Gly Leu Gly Thr Val Val Gly Leu Val
        355                 360                 365

Asn Lys Asp Leu Gly Lys Lys Leu Gly Ser Thr Val Gly Leu Val Ala
    370                 375                 380

Lys Asp Cys Ala Lys Lys Ile Val Ala Ser Ser Ala Met Gly Leu Val
385                 390                 395                 400

Asn Lys Asp Ile Gly Lys Lys Leu Met Ser Cys Pro Leu Ala Gly Leu
                405                 410                 415

Ile Ser Lys Asp Ala Ile Asn Leu Lys Ala Glu Ala Leu Leu Pro Thr
            420                 425                 430

Gln Glu Pro Leu Lys Ala Ser Cys Ser Thr Asn Ile Asn Asn Gln Glu
        435                 440                 445

Ser Gln Glu Leu Ser Glu Ser Leu Lys Asp Ser Ala Thr Ser Lys Thr
    450                 455                 460

Phe Glu Lys Asn Val Val Arg Gln Asn Lys Glu Ser Ile Leu Glu Lys
465                 470                 475                 480

Phe Ser Val Arg Lys Glu Ile Ile Asn Leu Glu Lys Glu Met Phe Asn
                485                 490                 495

Glu Gly Thr Cys Ile Gln Gln Asp Ser Phe Ser Ser Ser Glu Lys Gly
            500                 505                 510

Ser Tyr Glu Thr Ser Lys His Glu Lys Gln Pro Pro Val Tyr Cys Thr
        515                 520                 525

Ser Pro Asp Phe Lys Met Gly Gly Ala Ser Asp Val Ser Thr Ala Lys
    530                 535                 540

Ser Pro Phe Ser Ala Val Gly Glu Ser Asn Leu Pro Ser Pro Ser Pro
545                 550                 555                 560

Thr Val Ser Val Asn Pro Leu Thr Arg Ser Pro Pro Glu Thr Ser Ser
                565                 570                 575

Gln Leu Ala Pro Asn Pro Leu Leu Leu Ser Ser Thr Thr Glu Leu Ile
            580                 585                 590

Glu Glu Ile Ser Glu Ser Val Gly Lys Asn Gln Phe Thr Ser Glu Ser
        595                 600                 605

Thr His Leu Asn Val Gly His Arg Ser Val Gly His Ser Ile Ser Ile
    610                 615                 620

Glu Cys Lys Gly Ile Asp Lys Glu Val Asn Asp Ser Lys Thr Thr His
625                 630                 635                 640

Ile Asp Ile Pro Arg Ile Ser Ser Ser Leu Gly Lys Lys Pro Ser Leu
                645                 650                 655

Thr Ser Glu Ser Ser Ile His Thr Ile Thr Pro Ser Val Val Asn Phe
        660                 665                 670

Thr Ser Leu Phe Ser Asn Lys Pro Phe Leu Lys Leu Gly Ala Val Ser
    675                 680                 685

Ala Ser Asp Lys His Cys Gln Val Ala Glu Ser Leu Ser Thr Ser Leu
    690                 695                 700

Gln Ser Lys Pro Leu Lys Lys Arg Lys Gly Arg Lys Pro Arg Trp Thr
705                 710                 715                 720

Lys Val Val Ala Arg Ser Thr Cys Arg Ser Pro Lys Gly Leu Glu Leu
                725                 730                 735

Glu Arg Ser Glu Leu Phe Lys Asn Val Ser Cys Ser Ser Leu Ser Asn
            740                 745                 750

Ser Asn Ser Glu Pro Ala Lys Phe Met Lys Asn Ile Gly Pro Pro Ser
        755                 760                 765

Phe Val Asp His Asp Phe Leu Lys Arg Arg Leu Pro Lys Leu Ser Lys
    770                 775                 780

Ser Thr Ala Pro Ser Leu Ala Leu Leu Ala Asp Ser Glu Lys Pro Ser
785                 790                 795                 800

His Lys Ser Phe Ala Thr His Lys Leu Ser Ser Ser Met Cys Val Ser
                805                 810                 815

Ser Asp Leu Leu Ser Asp Ile Tyr Lys Pro Lys Arg Gly Arg Pro Lys
            820                 825                 830

Ser Lys Glu Met Pro Gln Leu Glu Gly Pro Pro Lys Arg Thr Leu Lys
        835                 840                 845

Ile Pro Ala Ser Lys Val Phe Ser Leu Gln Ser Lys Glu Glu Gln Glu
    850                 855                 860

Pro Pro Ile Leu Gln Pro Glu Ile Glu Ile Pro Ser Phe Lys Gln Gly
865                 870                 875                 880

Leu Ser Val Ser Pro Phe Pro Lys Lys Arg Gly Arg Pro Lys Arg Gln
                885                 890                 895

Met Arg Ser Pro Val Lys Met Lys Pro Pro Val Leu Ser Val Ala Pro
            900                 905                 910

Phe Val Ala Thr Glu Ser Pro Ser Lys Leu Glu Ser Glu Ser Asp Asn
        915                 920                 925

His Arg Ser Ser Ser Asp Phe Phe Glu Ser Glu Asp Gln Leu Gln Asp
    930                 935                 940

Pro Asp Asp Leu Asp Asp Ser His Arg Pro Ser Val Cys Ser Met Ser
945                 950                 955                 960

Asp Leu Glu Met Glu Pro Asp Lys Lys Ile Thr Lys Arg Asn Asn Gly
                965                 970                 975

Gln Leu Met Lys Thr Ile Ile Arg Lys Ile Asn Lys Met Lys Thr Leu
            980                 985                 990

Lys Arg Lys Lys Leu Leu Asn Gln  Ile Leu Ser Ser Ser  Val Glu Ser
        995                 1000                 1005

Ser Asn  Lys Gly Lys Val Gln  Ser Lys Leu His Asn  Thr Val Ser
    1010                 1015                 1020

Ser Leu  Ala Ala Thr Phe Gly  Ser Lys Leu Gly Gln  Gln Ile Asn
    1025                 1030                 1035

Val Ser  Lys Lys Gly Thr Ile  Tyr Ile Gly Lys Arg  Arg Gly Arg
    1040                 1045                 1050

Lys Pro  Lys Thr Val Leu Asn  Gly Ile Leu Ser Gly  Ser Pro Thr
    1055                 1060                 1065

Ser Leu  Ala Val Leu Glu Gln  Thr Ala Gln Gln Ala  Ala Gly Ser
    1070                 1075                 1080

Ala Leu  Gly Gln Ile Leu Pro  Pro Leu Leu Pro Ser  Ser Ala Ser
    1085                 1090                 1095

Ser Ser  Glu Ile Leu Pro Ser  Pro Ile Cys Ser Gln  Ser Ser Gly
    1100                 1105                 1110

Thr Ser  Gly Gly Gln Ser Pro  Val Ser Ser Asp Ala  Gly Phe Val
    1115                 1120                 1125

Glu Pro  Ser Ser Val Pro Tyr  Leu His Leu His Ser  Arg Gln Gly
    1130                 1135                 1140

Ser Met  Ile Gln Thr Leu Ala  Met Lys Lys Ala Ser  Lys Gly Arg

1145                1150                1155

Arg Arg Leu Ser Pro Pro Thr Leu Leu Pro Asn Ser Pro Ser His
    1160                1165                1170

Leu Ser Glu Leu Thr Ser Leu Lys Glu Ala Thr Pro Ser Pro Ile
    1175                1180                1185

Ser Glu Ser His Ser Asp Glu Thr Ile Pro Ser Asp Ser Gly Ile
    1190                1195                1200

Gly Thr Asp Asn Asn Ser Thr Ser Asp Arg Ala Glu Lys Phe Cys
    1205                1210                1215

Gly Gln Lys Lys Arg Arg His Ser Phe Glu His Val Ser Leu Ile
    1220                1225                1230

Pro Pro Glu Thr Ser Thr Val Leu Ser Ser Leu Lys Glu Lys His
    1235                1240                1245

Lys His Lys Cys Lys Arg Arg Asn His Asp Tyr Leu Ser Tyr Asp
    1250                1255                1260

Lys Met Lys Arg Gln Lys Arg Lys Arg Lys Lys Lys Tyr Pro Gln
    1265                1270                1275

Leu Arg Asn Arg Gln Asp Pro Asp Phe Ile Ala Glu Leu Glu Glu
    1280                1285                1290

Leu Ile Ser Arg Leu Ser Glu Ile Arg Ile Thr His Arg Ser His
    1295                1300                1305

His Phe Ile Pro Arg Asp Leu Leu Pro Thr Ile Phe Arg Ile Asn
    1310                1315                1320

Phe Asn Ser Phe Tyr Thr His Pro Ser Phe Pro Leu Asp Pro Leu
    1325                1330                1335

His Tyr Ile Arg Lys Pro Asp Leu Lys Lys Lys Arg Gly Arg Pro
    1340                1345                1350

Pro Lys Met Arg Glu Ala Met Ala Glu Met Pro Phe Met His Ser
    1355                1360                1365

Leu Ser Phe Pro Leu Ser Ser Thr Gly Phe Tyr Pro Ser Tyr Gly
    1370                1375                1380

Met Pro Tyr Ser Pro Ser Pro Leu Thr Ala Ala Pro Ile Gly Leu
    1385                1390                1395

Gly Tyr Tyr Gly Arg Tyr Pro Pro Thr Leu Tyr Pro Pro Pro Pro
    1400                1405                1410

Ser Pro Ser Phe Thr Thr Pro Leu Pro Pro Pro Ser Tyr Met His
    1415                1420                1425

Ala Gly His Leu Leu Leu Asn Pro Ala Lys Tyr His Lys Lys Lys
    1430                1435                1440

His Lys Leu Leu Arg Gln Glu Ala Phe Leu Thr Thr Ser Arg Thr
    1445                1450                1455

Pro Leu Leu Ser Met Ser Thr Tyr Pro Ser Val Pro Pro Glu Met
    1460                1465                1470

Ala Tyr Gly Trp Met Val Glu His Lys His Arg His Arg His Lys
    1475                1480                1485

His Arg Glu His Arg Ser Ser Glu Gln Pro Gln Val Ser Met Asp
    1490                1495                1500

Thr Gly Ser Ser Arg Ser Val Leu Glu Ser Leu Lys Arg Tyr Arg
    1505                1510                1515

Phe Gly Lys Asp Ala Val Gly Glu Arg Tyr Lys His Lys Glu Lys
    1520                1525                1530

His Arg Cys His Met Ser Cys Pro His Leu Ser Pro Ser Lys Ser
    1535                1540                1545

Leu Ile Asn Arg Glu Glu Gln Trp Val His Arg Glu Pro Ser Glu
    1550                1555                1560

Ser Ser Pro Leu Ala Leu Gly Leu Gln Thr Pro Leu Gln Ile Asp
    1565                1570                1575

Cys Ser Glu Ser Ser Pro Ser Leu Ser Leu Gly Gly Phe Thr Pro
    1580                1585                1590

Asn Ser Glu Pro Ala Ser Ser Asp Glu His Thr Asn Leu Phe Thr
    1595                1600                1605

Ser Ala Ile Gly Ser Cys Arg Val Ser Asn Pro Asn Ser Ser Gly
    1610                1615                1620

Arg Lys Lys Leu Thr Asp Ser Pro Gly Leu Phe Ser Ala Gln Asp
    1625                1630                1635

Thr Ser Leu Asn Arg Leu His Arg Lys Glu Ser Leu Pro Ser Asn
    1640                1645                1650

Glu Arg Ala Val Gln Thr Leu Ala Gly Ser Gln Pro Thr Ser Asp
    1655                1660                1665

Lys Pro Ser Gln Arg Pro Ser Glu Ser Thr Asn Cys Ser Pro Thr
    1670                1675                1680

Arg Lys Arg Ser Ser Ser Glu Ser Thr Ser Ser Thr Val Asn Gly
    1685                1690                1695

Val Pro Ser Arg Ser Pro Arg Leu Val Ala Ser Gly Asp Asp Ser
    1700                1705                1710

Val Asp Ser Leu Leu Gln Arg Met Val Gln Asn Glu Asp Gln Glu
    1715                1720                1725

Pro Met Glu Lys Ser Ile Asp Ala Val Ile Ala Thr Ala Ser Ala
    1730                1735                1740

Pro Pro Ser Ser Ser Pro Gly Arg Ser His Ser Lys Asp Arg Thr
    1745                1750                1755

Leu Gly Lys Pro Asp Ser Leu Leu Val Pro Ala Val Thr Ser Asp
    1760                1765                1770

Ser Cys Asn Asn Ser Ile Ser Leu Leu Ser Glu Lys Leu Thr Ser
    1775                1780                1785

Ser Cys Ser Pro His His Ile Lys Arg Ser Val Val Glu Ala Met
    1790                1795                1800

Gln Arg Gln Ala Arg Lys Met Cys Asn Tyr Asp Lys Ile Leu Ala
    1805                1810                1815

Thr Lys Lys Asn Leu Asp His Val Asn Lys Ile Leu Lys Ala Lys
    1820                1825                1830

Lys Leu Gln Arg Gln Ala Arg Thr Gly Asn Asn Phe Val Lys Arg
    1835                1840                1845

Arg Pro Gly Arg Pro Arg Lys Cys Pro Leu Gln Ala Val Val Ser
    1850                1855                1860

Met Gln Ala Phe Gln Ala Ala Gln Phe Val Asn Pro Glu Leu Asn
    1865                1870                1875

Arg Asp Glu Glu Gly Ala Ala Leu His Leu Ser Pro Asp Thr Val
    1880                1885                1890

Thr Asp Val Ile Glu Ala Val Val Gln Ser Val Asn Leu Asn Pro
    1895                1900                1905

Glu His Lys Lys Gly Leu Lys Arg Lys Gly Trp Leu Leu Glu Glu
    1910                1915                1920

Gln Thr Arg Lys Lys Gln Lys Pro Leu Pro Glu Glu Glu Glu Gln
    1925                1930                1935

Glu Asn Asn Lys Ser Phe Asn Glu Ala Pro Val Glu Ile Pro Ser
    1940                1945                1950

Pro Ser Glu Thr Pro Ala Lys Pro Ser Glu Pro Glu Ser Thr Leu
    1955                1960                1965

Gln Pro Val Leu Ser Leu Ile Pro Arg Glu Lys Lys Pro Pro Arg
    1970                1975                1980

Pro Pro Lys Lys Lys Tyr Gln Lys Ala Gly Leu Tyr Ser Asp Val
    1985                1990                1995

Tyr Lys Thr Thr Asp Pro Lys Ser Arg Leu Ile Gln Leu Lys Lys
    2000                2005                2010

Glu Lys Leu Glu Tyr Thr Pro Gly Glu His Glu Tyr Gly Leu Phe
    2015                2020                2025

Pro Ala Pro Ile His Val Val Phe Phe Val Ser Gly Lys Tyr Leu
    2030                2035                2040

Arg Gln Lys Arg Ile Asp Phe Gln Leu Pro Tyr Asp Ile Leu Trp
    2045                2050                2055

Gln Trp Lys His Asn Gln Leu Tyr Lys Lys Pro Asp Val Pro Leu
    2060                2065                2070

Tyr Lys Lys Ile Arg Ser Asn Val Tyr Val Asp Val Lys Pro Leu
    2075                2080                2085

Ser Gly Tyr Glu Ala Thr Thr Cys Asn Cys Lys Lys Pro Asp Asp

2090                2095                2100

Asp Thr Arg Lys Gly Cys Val Asp Asp Cys Leu Asn Arg Met Ile
    2105                2110                2115

Phe Ala Glu Cys Ser Pro Asn Thr Cys Pro Cys Gly Glu Gln Cys
    2120                2125                2130

Cys Asn Gln Arg Ile Gln Arg His Glu Trp Val Gln Cys Leu Glu
    2135                2140                2145

Arg Phe Arg Ala Glu Glu Lys Gly Trp Gly Ile Arg Thr Lys Glu
    2150                2155                2160

Pro Leu Lys Ala Gly Gln Phe Ile Ile Glu Tyr Leu Gly Glu Val
    2165                2170                2175

Val Ser Glu Gln Glu Phe Arg Asn Arg Met Ile Glu Gln Tyr His
    2180                2185                2190

Asn His Ser Asp His Tyr Cys Leu Asn Leu Asp Ser Gly Met Val
    2195                2200                2205

Ile Asp Ser Tyr Arg Met Gly Asn Glu Ala Arg Phe Ile Asn His
    2210                2215                2220

Ser Cys Asp Pro Asn Cys Glu Met Gln Lys Trp Ser Val Asn Gly
    2225                2230                2235

Val Tyr Arg Ile Gly Leu Tyr Ala Leu Lys Asp Met Pro Ala Gly
    2240                2245                2250

Thr Glu Leu Thr Tyr Asp Tyr Asn Phe His Ser Phe Asn Val Glu
    2255                2260                2265

Lys Gln Gln Leu Cys Lys Cys Gly Phe Glu Lys Cys Arg Gly Ile
    2270                2275                2280

Ile Gly Gly Lys Ser Gln Arg Val Asn Gly Leu Thr Ser Ser Lys
    2285                2290                2295

Asn Ser Gln Pro Met Ala Thr His Lys Lys Ser Gly Arg Ser Lys
    2300                2305                2310

Glu Lys Arg Lys Ser Lys His Lys Leu Lys Lys Arg Arg Gly His
    2315                2320                2325

Leu Ser Glu Glu Pro Ser Glu Asn Ile Asn Thr Pro Thr Arg Leu
    2330                2335                2340

Thr Pro Gln Leu Gln Met Lys Pro Met Ser Asn Arg Glu Arg Asn
    2345                2350                2355

Phe Val Leu Lys His His Val Phe Leu Val Arg Asn Trp Glu Lys
    2360                2365                2370

Ile Arg Gln Lys Gln Glu Glu Val Lys His Thr Ser Asp Asn Ile
    2375                2380                2385

His Ser Ala Ser Leu Tyr Thr Arg Trp Asn Gly Ile Cys Arg Asp
    2390                2395                2400

Asp Gly Asn Ile Lys Ser Asp Val Phe Met Thr Gln Phe Ser Ala
    2405                2410                2415

Leu Gln Thr Ala Arg Ser Val Arg Thr Arg Arg Leu Ala Ala Ala
    2420                2425                2430

Glu Glu Asn Ile Glu Val Ala Arg Ala Ala Arg Leu Ala Gln Ile
    2435                2440                2445

Phe Lys Glu Ile Cys Asp Gly Ile Ile Ser Tyr Lys Asp Ser Ser
    2450                2455                2460

Arg Gln Ala Leu Ala Ala Pro Leu Leu Asn Leu Pro Pro Lys Lys
    2465                2470                2475

Lys Asn Ala Asp Tyr Tyr Glu Lys Ile Ser Asp Pro Leu Asp Leu
    2480                2485                2490

Ile Thr Ile Glu Lys Gln Ile Leu Thr Gly Tyr Tyr Lys Thr Val
    2495                2500                2505

Glu Ala Phe Asp Ala Asp Met Leu Lys Val Phe Arg Asn Ala Glu
    2510                2515                2520

Lys Tyr Tyr Gly Arg Lys Ser Pro Val Gly Arg Asp Val Cys Arg
    2525                2530                2535

Leu Arg Lys Ala Tyr Tyr Asn Ala Arg His Glu Ala Ser Ala Gln
    2540                2545                2550

Ile Asp Glu Ile Val Gly Glu Thr Ala Ser Glu Ala Asp Ser Ser
    2555                2560                2565

Glu Thr Ser Val Ser Glu Lys Glu Asn Gly His Glu Lys Asp Asp
    2570                2575                2580

Asp Val Ile Arg Cys Ile Cys Gly Leu Tyr Lys Asp Glu Gly Leu
    2585                2590                2595

Met Ile Gln Cys Asp Lys Cys Met Val Trp Gln His Cys Asp Cys
    2600                2605                2610

Met Gly Val Asn Ser Asp Val Glu His Tyr Leu Cys Glu Gln Cys
    2615                2620                2625

Asp Pro Arg Pro Val Asp Arg Glu Val Pro Met Ile Pro Arg Pro
    2630                2635                2640

His Tyr Ala Gln Pro Gly Cys Val Tyr Phe Ile Cys Leu Leu Arg
    2645                2650                2655

Asp Asp Leu Leu Leu Arg Gln Gly Asp Cys Val Tyr Leu Met Arg
    2660                2665                2670

Asp Ser Arg Arg Thr Pro Asp Gly His Pro Val Arg Gln Ser Tyr
    2675                2680                2685

Arg Leu Leu Ser His Ile Asn Arg Asp Lys Leu Asp Ile Phe Arg
    2690                2695                2700

Ile Glu Lys Leu Trp Lys Asn Glu Lys Glu Glu Arg Phe Ala Phe
    2705                2710                2715

Gly His His Tyr Phe Arg Pro His Glu Thr His His Ser Pro Ser
    2720                2725                2730

Arg Arg Phe Tyr His Asn Glu Leu Phe Arg Val Pro Leu Tyr Glu
    2735                2740                2745

Ile Ile Pro Leu Glu Ala Val Val Gly Thr Cys Cys Val Leu Asp
    2750                2755                2760

Leu Tyr Thr Tyr Cys Lys Gly Arg Pro Lys Gly Val Lys Glu Gln
    2765                2770                2775

Asp Val Tyr Ile Cys Asp Tyr Arg Leu Asp Lys Ser Ala His Leu
    2780                2785                2790

Phe Tyr Lys Ile His Arg Asn Arg Tyr Pro Val Cys Thr Lys Pro
    2795                2800                2805

Tyr Ala Phe Asp His Phe Pro Lys Lys Leu Thr Pro Lys Lys Asp
    2810                2815                2820

Phe Ser Pro His Tyr Val Pro Asp Asn Tyr Lys Arg Asn Gly Gly
    2825                2830                2835

Arg Ser Ser Trp Lys Ser Glu Arg Ser Lys Pro Pro Leu Lys Asp
    2840                2845                2850

Leu Gly Gln Glu Asp Asp Ala Leu Pro Leu Ile Glu Glu Val Leu
    2855                2860                2865

Ala Ser Gln Glu Gln Ala Ala Asn Glu Ile Pro Ser Leu Glu Glu
    2870                2875                2880

Pro Glu Arg Glu Gly Ala Thr Ala Asn Val Ser Glu Gly Glu Lys
    2885                2890                2895

Lys Thr Glu Glu Ser Ser Gln Glu Pro Gln Ser Thr Cys Thr Pro
    2900                2905                2910

Glu Glu Arg Arg His Asn Gln Arg Glu Arg Leu Asn Gln Ile Leu
    2915                2920                2925

Leu Asn Leu Leu Glu Lys Ile Pro Gly Lys Asn Ala Ile Asp Val
    2930                2935                2940

Thr Tyr Leu Leu Glu Glu Gly Ser Gly Arg Lys Leu Arg Arg Arg
    2945                2950                2955

Thr Leu Phe Ile Pro Glu Asn Ser Phe Arg Lys
    2960                2965

<210>  23
<211>  481
<212>  PRT
<213>  Homo sapiens

<400>  23

Met Gly Lys Leu Arg Pro Gly Arg Val Glu Trp Leu Ala Ser Gly His
1               5                   10                  15

Thr Glu Arg Pro His Leu Phe Gln Asn Leu Leu Leu Phe Leu Trp Ala
            20                  25                  30

Leu Leu Asn Cys Gly Leu Gly Val Ser Ala Gln Gly Pro Gly Glu Trp
        35                  40                  45

Thr Pro Trp Val Ser Trp Thr Arg Cys Ser Ser Ser Cys Gly Arg Gly
    50                  55                  60

Val Ser Val Arg Ser Arg Arg Cys Leu Arg Leu Pro Gly Glu Glu Pro
65                  70                  75                  80

Cys Trp Gly Asp Ser His Glu Tyr Arg Leu Cys Gln Leu Pro Asp Cys
                85                  90                  95

Pro Pro Gly Ala Val Pro Phe Arg Asp Leu Gln Cys Ala Leu Tyr Asn
            100                 105                 110

Gly Arg Pro Val Leu Gly Thr Gln Lys Thr Tyr Gln Trp Val Pro Phe
        115                 120                 125

His Gly Ala Pro Asn Gln Cys Asp Leu Asn Cys Leu Ala Glu Gly His
    130                 135                 140

Ala Phe Tyr His Ser Phe Gly Arg Val Leu Asp Gly Thr Ala Cys Ser
145                 150                 155                 160

Pro Gly Ala Gln Gly Val Cys Val Ala Gly Arg Cys Leu Ser Ala Gly
                165                 170                 175

Cys Asp Gly Leu Leu Gly Ser Gly Ala Leu Glu Asp Arg Cys Gly Arg
            180                 185                 190

Cys Gly Gly Ala Asn Asp Ser Cys Leu Phe Val Gln Arg Val Phe Arg
        195                 200                 205

Asp Ala Gly Ala Phe Ala Gly Tyr Trp Asn Val Thr Leu Ile Pro Glu
    210                 215                 220

Gly Ala Arg His Ile Arg Val Glu His Arg Ser Arg Asn His Leu Ala
225                 230                 235                 240

Leu Met Gly Gly Asp Gly Arg Tyr Val Leu Asn Gly His Trp Val Val
                245                 250                 255

Ser Pro Pro Gly Thr Tyr Glu Ala Ala Gly Thr His Val Val Tyr Thr
            260                 265                 270

Arg Asp Thr Gly Pro Gln Glu Thr Leu Gln Ala Ala Gly Pro Thr Ser
        275                 280                 285

His Asp Leu Leu Leu Gln Val Leu Leu Gln Glu Pro Asn Pro Gly Ile

290                 295                 300

Glu Phe Glu Phe Trp Leu Pro Arg Glu Arg Tyr Ser Pro Phe Gln Ala
305                 310                 315                 320

Arg Val Gln Ala Leu Gly Trp Pro Leu Arg Gln Pro Gln Pro Arg Gly
                325                 330                 335

Val Glu Pro Gln Pro Pro Ala Ala Pro Ala Val Thr Pro Ala Gln Thr
            340                 345                 350

Pro Thr Leu Ala Pro Asp Pro Cys Pro Pro Cys Pro Asp Thr Arg Gly
        355                 360                 365

Arg Ala His Arg Leu Leu His Tyr Cys Gly Ser Asp Phe Val Phe Gln
    370                 375                 380

Ala Arg Val Leu Gly His His His Gln Ala Gln Glu Thr Arg Tyr Glu
385                 390                 395                 400

Val Arg Ile Gln Leu Val Tyr Lys Asn Arg Ser Pro Leu Arg Ala Arg
                405                 410                 415

Glu Tyr Val Trp Ala Pro Gly His Cys Pro Cys Pro Met Leu Ala Pro
            420                 425                 430

His Arg Asp Tyr Leu Met Ala Val Gln Arg Leu Val Ser Pro Asp Gly
        435                 440                 445

Thr Gln Asp Gln Leu Leu Leu Pro His Ala Gly Tyr Ala Arg Pro Trp
    450                 455                 460

Ser Pro Ala Glu Asp Ser Arg Ile Arg Leu Thr Ala Arg Arg Cys Pro
465                 470                 475                 480

Gly

<210>  24
<211>  416
<212>  PRT
<213>  Homo sapiens

<400>  24

<210>  25
<211>  369
<212>  PRT
<213>  Homo sapiens

<400>  25

Met Trp Cys Leu Glu Arg Leu Arg Leu Gly Pro Glu Cys Leu Arg Arg
1               5                   10                  15

Ser Gly Asp Trp Leu Leu Pro Gly Arg Ala Arg Gly Ala Lys Ser Arg
            20                  25                  30

Thr Thr Ala Ala Cys Ala Asn Val Leu Thr Pro Asp Arg Ile Pro Glu
        35                  40                  45

Phe Cys Ile Pro Pro Arg Leu Met Pro Arg Leu Ala Leu Ala Ala Leu
    50                  55                  60

Arg Asn Ser Trp Val Glu Glu Ala Gly Met Asp Glu Gly Ala Gly Arg
65                  70                  75                  80

Thr Asp Trp Asp Pro Arg Ser Gln Ala Ala Leu Ser Leu Pro His Leu
                85                  90                  95

Pro Arg Val Arg Thr Ala Tyr Gly Phe Cys Ala Leu Leu Glu Ser Pro
            100                 105                 110

His Thr Arg Arg Lys Glu Ser Leu Leu Leu Gly Gly Pro Pro Ala Pro
        115                 120                 125

Arg Pro Arg Ala His Thr Tyr Gly Gly Gly Gly Gly Pro Asp Ala Leu
    130                 135                 140

Leu Gly Thr Leu Arg Val Pro Arg Ala Pro Gly Pro Ala Thr Pro Ala
145                 150                 155                 160

Ala Pro Gly Cys Pro Arg Pro Pro Gln Asp Ala Leu Ala Arg Arg Pro
                165                 170                 175

Arg Gly Cys Arg Leu Leu Arg Val Pro Asp Gly Leu Leu Ser Arg Ala
            180                 185                 190

Leu Arg Ala Gly Arg Ser Arg Arg Leu Thr Arg Val Arg Ser Val Ser
        195                 200                 205

Ser Gly Asn Glu Asp Lys Glu Arg Arg Ala Gly Ser Gln Ser Pro Ala
    210                 215                 220

Arg Ala Pro Ser Thr Ser Pro Pro Ser Ser Arg Val Pro Phe Pro Glu
225                 230                 235                 240

Arg Leu Glu Ala Glu Gly Thr Val Ala Leu Gly Arg Ala Gly Asp Ala
                245                 250                 255

Leu Arg Leu Ala Ala Glu Tyr Cys Pro Gly Thr Gly Arg Leu Arg Leu
            260                 265                 270

Arg Leu Leu Arg Ala Glu Ser Pro Ala Gly Gly Ala Pro Gly Pro Arg
        275                 280                 285

Ala Val Ser Cys Arg Leu Ser Leu Val Leu Arg Pro Pro Gly Thr Ala
    290                 295                 300

Leu Arg Gln Cys Ser Thr Val Val Gly Arg Ser Arg Lys Ala Ser Phe
305                 310                 315                 320

Asp Gln Asp Phe Cys Phe Asp Gly Leu Ser Glu Asp Glu Val Arg Arg
                325                 330                 335

Leu Ala Val Arg Val Lys Ala Arg Asp Glu Gly Arg Gly Arg Glu Arg
            340                 345                 350

Gly Arg Leu Leu Gly Gln Gly Glu Leu Ser Leu Gly Ala Leu Leu Leu
        355                 360                 365

Leu

<210>  26
<211>  364
<212>  PRT
<213>  Homo sapiens

<400>  26

Met Arg Leu Leu Glu Lys Leu Cys Ser Ser Ala Ala Gly Ser Ser Ala
1               5                   10                  15

Pro Lys Pro Ala Phe Ala Lys Val Leu Thr Pro Asn Arg Ile Pro Glu
            20                  25                  30

Phe Cys Ile Pro Pro Arg Leu Pro Ala Pro Cys Thr Leu Glu Ser Pro
        35                  40                  45

Ile Arg Ala Ala Ala Val Pro Arg Arg Cys Ala Ala Glu Ser Asp Leu
    50                  55                  60

Trp Pro Arg Ala Ala Asp Glu Asp Ala Gly Arg Thr Asp Trp Asp Pro
65                  70                  75                  80

Arg Ser Gln Ala Ala Leu Ser Leu Pro His Leu Pro Arg Val Arg Thr
                85                  90                  95

Thr Tyr Gly Phe Cys Ala Leu Leu Glu Ser Pro His Thr Arg Arg Lys
            100                 105                 110

Glu Ser Leu Leu Leu Gly Gly Pro Pro Ala Pro Arg Pro Arg Ala His
        115                 120                 125

Ser Cys Gly Gly Gly Gly Gly Pro Asp Ala Pro Leu Gly Thr Leu Cys
    130                 135                 140

Gly Pro Arg Gly Pro Gly Pro Ala Thr Pro Ala Ala Pro Gly Gly Pro
145                 150                 155                 160

Arg Leu Pro Gln Asp Ala Leu Ala Ala Gly Pro Arg Arg Cys Arg Leu
                165                 170                 175

Leu Arg Val Pro Asp Gly Leu Leu Ser Arg Ala Leu Arg Ala Gly Arg
            180                 185                 190

Ser Arg Arg Leu Ala Arg Val Arg Ser Val Ser Ser Gly Asn Glu Asp
        195                 200                 205

Glu Glu Arg Arg Ala Gly Ser Glu Ser Pro Ala Arg Ala Pro Ser Ser
    210                 215                 220

Ser Pro Leu Ser Ser Arg Ala Pro Leu Pro Glu Arg Leu Glu Ala Lys
225                 230                 235                 240

Gly Thr Val Ala Leu Gly Arg Ala Gly Asp Ala Leu Arg Leu Ala Ala
                245                 250                 255

Glu Tyr Cys Pro Gly Thr Arg Arg Leu Arg Leu Arg Leu Leu Arg Ala
            260                 265                 270

Glu Ser Leu Phe Gly Gly Ala Pro Gly Pro Arg Ala Val Arg Cys Arg
        275                 280                 285

Leu Ser Leu Val Leu Arg Pro Pro Gly Thr Ala Arg Trp Gln Cys Ser
    290                 295                 300

Ala Val Val Gly Arg Ser Arg Lys Ala Ser Phe Asp Gln Asp Phe Cys
305                 310                 315                 320

Phe Asp Gly Leu Ser Glu Asp Glu Val Arg Arg Leu Ala Val Arg Val
                325                 330                 335

Lys Ala Arg Asp Glu Gly Arg Gly Arg Asp Arg Gly Arg Leu Leu Gly
            340                 345                 350

Gln Gly Glu Leu Ser Leu Gly Ala Leu Leu Leu Leu
        355                 360

<210> 27
<211> 208
<212> PRT
<213> Homo sapiens

<400> 27

Met Leu Gly Leu Leu Val Ala Leu Leu Ala Leu Gly Leu Ala Val Phe
1               5                   10                  15

Ala Leu Leu Asp Val Trp Tyr Leu Val Arg Leu Pro Cys Ala Val Leu
            20                  25                  30

Arg Ala Arg Leu Leu Gln Pro Arg Val Arg Asp Leu Leu Ala Glu Gln
        35                  40                  45

Arg Phe Pro Gly Arg Val Leu Pro Ser Asp Leu Asp Leu Leu Leu His
    50                  55                  60

Met Asn Asn Ala Arg Tyr Leu Arg Glu Ala Asp Phe Ala Arg Val Ala
65                  70                  75                  80

His Leu Thr Arg Cys Gly Val Leu Gly Ala Leu Arg Glu Leu Arg Ala
                85                  90                  95

His Thr Val Leu Ala Ala Ser Cys Ala Arg His Arg Arg Ser Leu Arg
            100                 105                 110

Leu Leu Glu Pro Phe Glu Val Arg Thr Arg Leu Leu Gly Trp Asp Asp
        115                 120                 125

Arg Ala Phe Tyr Leu Glu Ala Arg Phe Val Ser Leu Arg Asp Gly Phe
    130                 135                 140

Val Cys Ala Leu Leu Arg Phe Arg Gln His Leu Leu Gly Thr Ser Pro
145                 150                 155                 160

Glu Arg Val Val Gln His Leu Cys Gln Arg Arg Val Glu Pro Pro Glu
                165                 170                 175

Leu Pro Ala Asp Leu Gln His Trp Ile Ser Tyr Asn Glu Ala Ser Ser
            180                 185                 190

Gln Leu Leu Arg Met Glu Ser Gly Leu Ser Asp Val Thr Lys Asp Gln
        195                 200                 205

<210>  28
<211>  507
<212>  PRT
<213>  Homo sapiens

<400>  28

Met Asp Pro Ser Glu Lys Lys Ile Ser Val Trp Ile Cys Gln Glu Glu
1               5                   10                  15

Lys Leu Val Ser Gly Leu Ser Arg Arg Thr Thr Cys Ser Asp Val Val
            20                  25                  30

Arg Val Leu Leu Glu Asp Gly Cys Arg Arg Arg Arg Arg Gln Arg Arg
        35                  40                  45

Ser Arg Arg Leu Gly Ser Ala Gly Asp Pro His Gly Pro Gly Glu Leu
    50                  55                  60

Pro Glu Pro Pro Asn Glu Asp Asp Glu Asp Asp Asp Glu Ala Leu Pro
65                  70                  75                  80

Gln Gly Met Leu Cys Gly Pro Pro Gln Cys Tyr Cys Ile Val Glu Lys
                85                  90                  95

Trp Arg Gly Phe Glu Arg Ile Leu Pro Asn Lys Thr Arg Ile Leu Arg
            100                 105                 110

Leu Trp Ala Ala Trp Gly Glu Glu Gln Glu Asn Val Arg Phe Val Leu
        115                 120                 125

Val Arg Ser Glu Ala Ser Leu Pro Asn Ala Gly Pro Arg Ser Ala Glu
    130                 135                 140

Ala Arg Val Val Leu Ser Arg Glu Arg Pro Cys Pro Ala Arg Gly Ala
145                 150                 155                 160

Pro Ala Arg Pro Ser Leu Ala Met Thr Gln Glu Lys Gln Arg Arg Val
                165                 170                 175

Val Arg Lys Ala Phe Arg Lys Leu Ala Lys Leu Asn Arg Arg Arg Gln
            180                 185                 190

Gln Gln Thr Pro Ser Ser Cys Ser Ser Thr Ser Ser Ser Thr Ala Ser
        195                 200                 205

Ser Cys Ser Ser Ser Pro Arg Thr His Glu Ser Ala Ser Val Glu Arg
    210                 215                 220

Met Glu Thr Leu Val His Leu Val Leu Ser Gln Asp His Thr Ile Arg
225                 230                 235                 240

Gln Gln Val Gln Arg Leu His Glu Leu Asp Arg Glu Ile Asp His Tyr
                245                 250                 255

Glu Ala Lys Val His Leu Asp Arg Met Arg Arg His Gly Val Asn Tyr
            260                 265                 270

Val Gln Asp Thr Tyr Leu Val Gly Ala Gly Ile Glu Leu Asp Gly Ser
        275                 280                 285

Arg Pro Gly Glu Glu Pro Glu Glu Val Ala Ala Glu Ala Glu Glu Ala
    290                 295                 300

Ala Ala Ala Pro Pro Leu Ala Gly Glu Ala Gln Ala Ala Ala Leu Glu
305                 310                 315                 320

Glu Leu Ala Arg Arg Cys Asp Asp Leu Leu Arg Leu Gln Glu Gln Arg
                325                 330                 335

Val Gln Gln Glu Glu Leu Leu Glu Arg Leu Ser Ala Glu Ile Gln Glu
            340                 345                 350

Glu Leu Asn Gln Arg Trp Met Arg Arg Arg Gln Glu Glu Leu Ala Ala
        355                 360                 365

Arg Glu Glu Pro Leu Glu Pro Asp Gly Gly Pro Asp Gly Glu Leu Leu
    370                 375                 380

Leu Glu Gln Glu Arg Val Arg Thr Gln Leu Ser Thr Ser Leu Tyr Ile
385                 390                 395                 400

Gly Leu Arg Leu Asn Thr Asp Leu Glu Ala Val Lys Ser Asp Leu Asp
                405                 410                 415

Tyr Ser Gln Gln Gln Trp Asp Ser Lys Lys Arg Glu Leu Gln Gly Leu
            420                 425                 430

Leu Gln Thr Leu His Thr Leu Glu Leu Thr Val Ala Pro Asp Gly Ala
        435                 440                 445

Pro Gly Ser Gly Ser Pro Ser Arg Glu Pro Gly Pro Gln Ala Cys Ala
    450                 455                 460

Asp Met Trp Val Asp Gln Ala Arg Gly Leu Ala Lys Ser Gly Pro Gly
465                 470                 475                 480

Asn Asp Glu Asp Ser Asp Thr Gly Leu Ser Ser Met His Ser Gln Asp
                485                 490                 495

Ser Asp Ser Leu Pro Met Cys Glu Ser Leu Val
            500                 505

<210>  29
<211>  51
<212>  PRT
<213>  Homo sapiens

<400>  29

<210>  30
<211>  101
<212>  PRT
<213>  Homo sapiens

<400>  30

Gly Cys Glu Thr Pro Arg Leu Pro Glu Ile Ala Leu Arg Ala Leu Pro
1               5                   10                  15

Arg Pro Gly Val Ser Ser Leu Gly Thr Ile Leu Cys Val Pro Ser Val
            20                  25                  30

Pro Val Gly Pro Ile Val Leu Thr Ser Ala Thr Pro Glu Ser Ser Arg
        35                  40                  45

Phe Thr Arg Pro Ala Pro Ser Thr Val Pro Glu Ala His Arg His Ser
    50                  55                  60

Arg Ile Leu Arg Leu Ala Pro Val Pro Cys Arg Cys Tyr Arg Pro Pro
65                  70                  75                  80

Ala Pro Ala His Thr Ser Arg Ser Thr Ser Leu Phe Tyr Glu Ser Pro
                85                  90                  95

Ser Thr Phe Asn Val
            100

<210>  31
<211>  356
<212>  PRT
<213>  Homo sapiens

<400>  31

Met Gly Leu Met Val Arg Gly Gly Val Thr Cys Arg Ser Gln Glu Arg
1               5                   10                  15

Leu Leu Gly Val Gly Thr Gly Thr Ala Ser Ala Gln Pro Pro Val Thr
            20                  25                  30

Ala Ser Pro Tyr Cys Ser Phe Thr Gly Arg Ser Arg Ala Asp Ala Pro
        35                  40                  45

Ala Arg Ala Ala Gly Asn Gly Gln Pro Arg Leu Arg Ala Gln Val Asp
    50                  55                  60

Leu Ser Ser Ala Ser Ser Val Leu Val Thr Val Leu Asp Ala Lys Ala
65                  70                  75                  80

Leu Pro Leu Thr Leu Leu Ser Trp Gly Ser Leu Cys Ala Thr Ala Gln
                85                  90                  95

Asn His Asn Asn Gln Glu Gly Ala Pro Ala Gln Pro Lys Gly Ser Met
            100                 105                 110

Ala Asn Gln Arg Ala Leu Arg Phe Pro Pro Pro Ser Gly Ser Pro Pro
        115                 120                 125

Gly Leu His Thr Arg Met Arg Leu Thr Ser Pro Pro Gln Pro His Phe
    130                 135                 140

Glu Pro Pro Pro Pro Thr Thr Val Gly Ala Pro Arg Ser Leu Gln Gly
145                 150                 155                 160

Asp Trp Phe Gly Ser Asp Ser Arg Ala Val Arg Ala Leu Arg Leu Ile
                165                 170                 175

Gly Trp Ala Ser Arg Ser Leu His Pro Leu Pro Gly Ser Arg Asp Arg
            180                 185                 190

Ala His Pro Ala Ala Glu Glu Glu Asp Asp Pro Asp Arg Pro Ile Glu
        195                 200                 205

Phe Ser Ser Ser Lys Ala Asn Pro His Arg Trp Ser Val Gly His Thr
    210                 215                 220

Met Gly Lys Gly His Gln Arg Pro Trp Trp Lys Val Leu Pro Leu Ser
225                 230                 235                 240

Cys Phe Leu Val Ala Leu Ile Ile Trp Cys Tyr Leu Arg Glu Glu Ser
                245                 250                 255

Glu Ala Asp Gln Trp Leu Arg Gln Glu Gly Ser Arg Gln Pro Pro Phe
            260                 265                 270

Gly Phe Asp Val Thr Phe Ala Arg Asp Cys Pro Gly Tyr Ala Cys Val
        275                 280                 285

Leu Ser Thr Glu Gly Leu Gly Trp Trp Met Gly His Leu Ala Met Leu
    290                 295                 300

Ile Arg Val Lys Ala Glu Gln Asn Leu Ser Arg Ser Glu Thr Ala Pro
305                 310                 315                 320

Arg Leu Ala Leu Asp Val Gln Gly Phe His Arg Gln Asp Phe Ser Asp
                325                 330                 335

Pro Trp Gly Arg Phe Gln Leu His Cys Met Leu Leu Asp Leu Pro Ser
            340                 345                 350

Leu Cys Ile Thr
        355

## Description

The present invention relates to peptides with a conserved amino acid motif and pharmaceutical compositions comprising these peptides. The present invention further relates to the use of the peptides as biomarker, the medical use of the peptides, in particular in the diagnosis, prevention, monitoring and/or treatment of Psoriasis. The present invention relates to complexes of the peptides of the invention with HLA-C monomers or multimers and their use as biomarker and their medical uses, in particular in the treatment of Psoriasis and in monitoring such treatment. The present invention furthermore relates to means and methods for the prevention and/or treatment of Psoriasis, that comprise inhibiting or blocking the interaction of TCR and HLA-C.

### BACKGROUND OF THE INVENTION

Biochemical interactions between peptide epitope, specific membrane molecules encoded by the Major Histocompatibility Complex (MHC, in humans human leukocyte antigens HLA) and T-cell antigen receptors (TCR) are required to elicit specific immune responses. This requires activation of T cells by presentation to the T cells of peptides against which a T-cell response should be raised. The peptides are presented to the T-cells by HLA-molecules. T-cells express a clonotypic TCR on the surface. This receptor enables the T-cell to recognize peptide antigens bound to HLA molecules in man. Depending on the type of antigen, being intracellular or extracellular, the antigenic peptides are bound to MHC class I or MHC class II, respectively. The two classes of HLA complexes are recognized by different subsets of T-cells; cytotoxic CD8⁺ T-cells recognizing HLA-class I and CD4⁺ helper cells recognizing HLA-class II. In general, TCR recognition of MHC-peptide complexes results in T-cell activation, clonal expansion and differentiation of the T-cells into effector, memory and regulatory T-cells.

Several autoimmune diseases are associated with particular HLA-class I alleles. Deciphering the specificity of autoimmune responses and understanding the role of the human leukocyte antigen (HLA) region in autoimmune diseases, with different allelic variants associated with each disease, are important unmet medical needs. Neither the functional implications of the HLA associations nor specific antigens of autoreactive T-cell responses have been determined with certainty. This is due to the complexity of antigen-presentation and TCR-antigen recognition. Antigens of CD8⁺ T cells are short peptides of 8-10 amino acids that are cleaved from intracellular proteins by the proteasome and loaded onto HLA-class I molecules. The peptide-HLA-class complex is then transported onto the cell surface for cognate antigen recognition by αβ TCRs of CD8⁺ T cells (Neefjes et al., 2011). αβ TCRs are heterodimeric molecules composed of an α- and β-chain that are generated by somatic recombination of diverse gene segments and allow for a huge TCR diversity (Davis and Bjorkman, 1988). Approaches to characterize targets of autoimmune responses have to identify and utilize the relevant pathogenic autoreactive TCRs; determine, which HLA molecule of each two HLA-A, -B or -C alleles actually restricts the autoreactive T-cell response; define the autoimmune target cell type within the affected tissue; and provide a test system for the reliable identification and verification of the appropriate autoantigenic TCR peptide ligand. In their entirety, the challenges associated herewith are hard to master.

Psoriasis, or more precisely psoriasis vulgaris is a frequent immune-mediated HLA-associated inflammatory skin disease affecting as presumed, 120 to 180 million people worldwide (Icen et al., 2009; Parisi et al., 2013). Psoriasis has various clinical manifestations, including but not limited to chronic plaque psoriasis, acute guttate psoriasis, localized or generalized pustular psoriasis, psoriatic arthritis, psoriatic nail disease and others. Psoriatic skin lesions are characterized by an excessive inflammatory hyperplasia of the epidermis, which leads to the formation of typical red and scaly plaques (Griffiths and Barker, 2007). Many psoriasis features are consistent with a T-cell mediated autoimmune pathogenesis (Davidson and Diamond, 2001). Like other autoimmune diseases (Uchansk-Ziegler et al., 2012), psoriasis is linked to a particular HLA class I allele: The HLA-class I allele, HLA-C*06:02, on psoriasis susceptibility 1 locus (PSORS1, [MIM177900]) is a major psoriasis risk gene (Nair et al., 2006). The formation of novel psoriatic lesions depends on epidermal influx (Conrad et al., 2007) and clonal expansion of CD8⁺ T cells (Kim et al., 2012; Chang et al., 1997), which cause the epidermal changes by a T helper/suppressor (Th/Ts) 17-like effector phenotype (DiCesare et al., 2009). The most efficient treatments for psoriasis are immunosuppressive, interfere with T-cell recruitment and activation, or neutralize Th17-related cytokines (Griffiths and Barker, 2007; Chiricozzi and Krueger, 2013). Nevertheless, the mechanisms that lead to an organ-specific pathogenic T-cell response in psoriasis are unknown and the autoimmune nature of psoriasis remains hypothetical to date.

Thus, there is a need in the art for improved means and methods for diagnosing and treating Psoriasis, in particular for monitoring the treatment.

### SUMMARY OF THE INVENTION

According to the present invention this object is solved by a a peptide comprising an amino acid sequence of the general formula I

**X1ₙ - Arg - X2 - X3 - Y1 - X4 - Y2 - Arg - Z** (I)

wherein
- **X1**: is selected from Phe, Arg, Gly, Met, Ala, Ser, Leu or Val;
- **n**: is 0 or 1.
- **X2**: is selected from any amino acid;
- **X3**: is selected from any amino acid;
- **X4**: is selected from Thr, Tyr, Val, Cys, Ser, Ala;
- **Y1**: is selected from Arg, Lys, Gln, or Asn;
- **Y2**: is selected from Arg, Leu or Ser; and
- **Z**: is selected from Leu, Met, Ile or Val.

According to the present invention this object is solved by a peptide- HLA-C monomer complex,
comprising
(a) at least one peptide of the present invention,
(b) a HLA-C monomer binding to T-cell receptor(s) (TCR(s)).

According to the present invention this object is solved by a peptide- HLA-C multimer complex,
comprising a HLA-C multimer which comprises at least two HLA-C monomers (b) and which each comprises at least one peptide of the present invention.

According to the present invention this object is solved by a pharmaceutical composition, comprising
(i) at least one peptide of the present invention and/or a HLA-C monomer as defined herein;
   or a peptide- HLA-C monomer complex of the present invention,
   or a peptide- HLA-C multimer complex of the present invention,
(ii) optionally, a pharmaceutically acceptable carrier and/or excipient.

According to the present invention this object is solved by using a peptide of the present invention or a peptide- HLA-C monomer complex of the present invention or a peptide-HLA-C multimer complex of the present invention or a pharmaceutical composition of the present invention or a protein comprising an amino acid sequence of SEQ ID NOs. 19 to 27 as biomarker for Psoriasis, preferably Psoriasis vulgaris.

According to the present invention this object is solved by providing a peptide of the present invention or a peptide- HLA-C monomer complex of the present invention or a peptide-HLA-C multimer complex of the present invention or a pharmaceutical composition of the present invention for use in medicine.

According to the present invention this object is solved by providing a peptide of the present invention or a peptide- HLA-C monomer complex of the present invention or a peptide-HLA-C multimer complex of the present invention or a pharmaceutical composition of the present invention or a protein comprising an amino acid sequence of SEQ ID NOs. 19 to 27 for use in the diagnosis, prevention, monitoring and/or treatment of Psoriasis, preferably Psoriasis vulgaris.

According to the present invention this object is solved by a method for the diagnosis, prevention, monitoring and/or treatment of Psoriasis, preferably Psoriasis vulgaris, comprising the step of administering a therapeutically effective amount of at least one peptide of the present invention or a peptide- HLA-C monomer complex of the present invention or a peptide-HLA-C multimer complex of the present invention or a pharmaceutical composition of the present invention to a subject in need thereof.

According to the present invention this object is solved by providing a compound that is directed against HLA-C for use in the prevention, and/or treatment of Psoriasis, preferably Psoriasis vulgaris,
wherein said compound is selected from an anti-HLA-C antibody or fragment thereof (such as Fab, Fv, scFv), a small molecule (inhibitor), a small interfering RNA.

According to the present invention this object is solved by a method for the prevention and/or treatment of Psoriasis, preferably Psoriasis vulgaris, comprising
(a) inhibiting or blocking the interaction of TCR and HLA-C, and/or
(b) suppressing the expression of HLA-C.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "8 to 150 amino acids" should be interpreted to include not only the explicitly recited values of 8 to 150, but also include individual values and sub-ranges within the indicated range. Thus, included in this numerical range are individual values such as 8, 9, 10, 11, 12, 13, 14, 15, 16, 17, 18, 19, 20, 21, 22, 23, 24, 25, 26, 27, 28, 29 ,... 147, 148, 150 and sub-ranges such as from 8 to 100, from 9 to 50, from 9 to 15, from 8 to 00, etc. This same principle applies to ranges reciting only one numerical value, such as "at least 9 amino acids" or "at least 8 amino acids" or "up to 12 amino acids". Furthermore, such an interpretation should apply regardless of the breadth of the range or the characteristics being described.

### Antigenic peptides

As discussed above, the present invention provides antigenic peptides.

A peptide according to the present invention comprises or consists of an amino acid sequence of the general formula I

**X1ₙ - Arg - X2 - X3 - Y1 - X4 - Y2 - Arg - Z** **(I)**

wherein
- **X1**: is selected from Phe, Arg, Gly, Met, Ala, Ser, Leu or Val;
- **n**: is 0 or 1.
- **X2**: is selected from any amino acid;
- **X3**: is selected from any amino acid;
- **X4**: is selected from Thr, Tyr, Val, Cys, Ser, Ala;
- **Y1**: is selected from Arg, Lys, Gln, or Asn;
- **Y2**: is selected from Arg, Leu or Ser; and
- **Z**: is selected from Leu, Met, Ile or Val.

Preferably, the peptides of the present invention have a length of at least 9 amino acids.

In one embodiment, the peptides of the present invention have a length of at least 8 amino acids. In this embodiment, the peptide does not comprise amino acid X1, i.e. n = 0.

Preferably, the peptides of the present invention have a length of at least 8 or 9 amino acids,
preferably about 8 to 150 amino acids or about 9 to 150 amino acids.
more preferably about 8 to 100 amino acids or about 9 to 100 amino acids,
more preferably about 8 to 50 or about 9 to 50 amino acids,
more preferably about 8 to 20 or about 9 to 20 amino acids,
even more preferably about 8 to 15 or about 9 to 15 amino acids, such as up to 12 or 13 amino acids;

The amino acids of the peptides of the present invention can be L-amino acids or D-amino acids; peptides of the present invention comprise amino acids that are either L-amino acids or D-amino acids.

In one embodiment, a peptide of the present invention comprises further component(s), which is/are preferably selected from
N- and/or C-terminal modification(s)
such as comprising acetylation and/or amidation of the *N-* and/or *C*-terminus, label(s),
such as fluorescent dye(s), radioisotope(s) and/or contrast agent(s); tag(s);
drug(s) or their respective prodrug(s);
carrier or depot(s) for drug(s), prodrug(s) or label(s);
immunogenic epitope(s);
cytotoxin(s),
such as saporin, gelonin or ricin;
radionuclides,
such as actinium-225, astatine-211, bismuth-213, iodide-131, optionally with a chelate group
and/or combinations thereof.

The further component(s) is/are preferably covalently attached, such as via an amino acid side chain, a linker, spacer and/or anchor group(s), e.g. a cleavable linker.

Preferably, the peptide of the present invention comprises or is an antigen causing a T cell triggered immune response, preferably a CD8⁺ T cell antigen.

In one embodiment, the peptide of the present invention comprises or is an autoantigen causing a T cell triggered immune response, preferably a CD8⁺ T cell autoantigen.

An "autoantigen" or "autoantigenic peptide" or "self-antigen" as used herein refers to an endogenous tissue constituent that stimulates production of autoantibodies or self-reactive T lymphocytes in the subject in which it occurs and thus stimulates a humoral or cell-mediated immune response against self.

In a preferred embodiment, the peptide of the present invention is a peptide, wherein
- **X2**: is selected from Ser, His, Cys, Trp, Asn, Ala, Tyr, Gln, or Phe;
such as
selected from Ser, His, Cys, Trp, Asn, Ala, or Tyr; or
selected from His, Gln, Ser, Ala or Phe;
and/or
- **X3**: is selected from Tyr, Arg, Trp, Ser, Ala, His, Phe, Val, Gly, or Cys;
such as
selected from Tyr, Arg, Trp, Ser, Ala, His, Phe, or Ala; or
selected from Val, Gly, Arg, Ser or Cys.

In a preferred embodiment, the peptide of the present invention is a peptide, wherein
- **X1,**: if present, is selected from Phe, Arg, Gly, Met, Ala, Ser, Leu or Val;
such as
selected from Phe, Arg, Gly, or Met; or
selected from Ala, Arg, Ser or Leu;
and/or
- **X4**: is selected from Thr, Tyr, Val, Cys, Ser, Ala;
such as
selected from Thr, Tyr, Val, or Cys; or
selected from Ser, Ala, Cys or Thr;
and/or
- **Y2**: is selected from Arg, Leu or Ser; and
such as
selected from Arg, or Leu; or
selected from Arg, Leu or Ser.

In a preferred embodiment, the peptide of the present invention is a peptide, wherein
- **Z**: is Leu; or
- **Y2**: is Arg; or
- **Y2**: is Arg and Z is Leu, Met, Ile or Val; or
- **Y2**: is Arg and Z is Leu;
preferably comprising an amino acid sequence selected from the following formulas

**X1 - Arg - X2 - X3 - Y1 - X4 - Y2 - Arg - Leu** (Ia)

**X1 - Arg - X2 -X3 - Y1 - X4 - Arg - Arg - Z** (Ib)

**X1 - Arg - X2 - X3 - Y1 - X4 - Arg - Arg - Leu** (Ia)

wherein
- **X1, X2, X3, X4 Y1**: and are as herein above;
- **Y2**: is selected from Arg, or Leu;
- **Z**: is selected from **Z** is Leu, Met, Ile or Val, preferably Leu or Met.

In one embodiment, the peptides of the present invention are mimotopes.

A "mimotope" as used herein refers to an artificial peptide that acts as a TCR ligand and thus mimics a natural peptide epitope from proteins, carbohydrates or glycolipids.

Preferably, a peptide of the present invention comprises or consists of an amino acid sequence selected from SEQ ID NOs. 1 to 9.

| SEQ ID NO. 1 | FRSYRTRRL |
|---|---|
| 2 | FRHRRTLRL |
| 3 | FRCRQYRRL |
| 4 | FRWYRVRRL |
| 5 | RRNWNYRRL |
| 6 | GRASRTRRL |
| 7 | GRNARVRRL |
| 8 | MRSHRYLRL |
| 9 | RRYFRCLRM |

In one embodiment, the peptides of the present invention are natural ligands of proteins.

Preferably, a peptide of the present invention comprises or consists of an amino acid sequence selected from SEQ ID NOs. 10 to 19.

| SEQ ID NO. | | |
|---|---|---|
| 10 | ARHRRSLRL | THEM6 |
| 11 | RRQRRSRRL | RASSF10 |
| 12 | RQRRSRRL | RASSF10 |
| 13 | ARSVRTRRL | ASH1L |
| 14 | SRASRALRL | Hepacam |
| 15 | LRAGRSRRL | C2CD4A/B |
| 16 | RAGRSRRL | C2CD4A/B |
| 17 | VRSRRCLRL | ADAMTL5 |
| 18 | RSRRCLRL | ADAMTL5 |
| 19 | RRFCRTSRM | IL24delE3 |
| 20 | HRHSRILRL | HSPC088 |
| 21 | SRAVRALRL | |

For further details, see also Table 2 below.

### Peptide- HLA-C complexes

As discussed above, the present invention provides complexes of the antigenic peptides of the present invention with HLA-C.

HLA-C belongs to the MHC (human = HLA) class I heavy chain receptors. The C receptor is a heterodimer consisting of a HLA-C mature gene product and β2-microglobulin. The mature C chain is anchored in the cell membrane. MHC Class I molecules, like HLA-C, are expressed by all nucleated cells, and present small peptides to the immune system which surveys for peptide antigens derived from autologous or non-self proteins.

The complexes of the present invention can, for example, be obtained *in vitro* from a recombinant HLA-C which folds back in presence of the peptide of the present invention.

A peptide- HLA-C monomer complex according to the present invention comprises
(a) at least one peptide of the present invention,
(b) a HLA-C monomer binding to T-cell receptor(s) (TCR(s)).

A peptide- HLA-C multimer complex according to the present invention comprises a HLA-C multimer which comprises at least two HLA-C monomers defined as (b) herein and which each comprises at least one peptide of the present invention.

In one embodiment, the HLA-C comprises of the monomer or multimer complexes of the present invention comprises further component(s), such as
label(s),
such as fluorescent dye(s), radioisotope(s) and/or contrast agent(s);
tag(s);
drug(s) or their respective prodrug(s);
carrier or depot(s) for drug(s), prodrug(s) or label(s);
immunogenic epitope(s);
cytotoxin(s),
such as saporin, gelonin or ricin;
radionuclides,
such as actinium-225, astatine-211, bismuth-213, iodide-131, optionally with a chelate group
coupling moiety/moieties;
and/or combinations thereof.

The further component(s) is/are preferably covalently attached, such as via an amino acid side chain, a linker, spacer and/or anchor group(s),
e.g. a cleavable linker.

Preferably, the coupling moiety/moieties are comprised in the HLA-C monomer and are suitable for obtaining HLA-C oligomers or multimers.

Examples for such coupling moiety/moieties are

### (1) Dextrane

Usually 10 to 25 HLA-C monomers covalently attach to one dextrane molecule (and form a so-called dextramer). See e.g. Scholler et al., 2010.

### (2) Biotin

The multimerization can e.g. be obtained via the well-known interaction of biotin with streptavidin or avidin.

In one embodiment, the HLA-C comprises biotin as coupling moiety which then interacts with streptavidin or avidin that can e.g. be attached to a surface, such as in a microtiter plate/well or the like.

For example, the most commonly used MHC or HLA-C multimers are tetramers. These are typically produced by biotinylating soluble MHC or HLA-C monomers, which are typically produced recombinantly in eukaryotic or bacterial cells. These monomers then bind to a backbone, such as streptavidin or avidin, creating a tetravalent structure. These backbones can be further conjugated with fluorochromes and can be used to subsequently isolate bound T-cells via flow cytometry.
See e.g. Altmann et al., 1996.

### (3) IgG-Fc regions

The HLA-C chain can be linked to the IgG-Fc region of immunoglobulins, creating HLA-C-IgG-Fc fusion proteins.

Preferred HLA-C multimers in the peptide-HLA-C multimer complexes of the present invention are comprised of 2, 3, 4, 5, 6, 7, 8, 9, 10 or more HLA-C monomers, such as dimers, trimmers, tetramers, pentamers, octamers, and the like.

In one embodiment, oligomers with a distribution of monomers per aggregates are comprised in the peptide-HLA-C multimer complexes of the present invention. For example, in case of "dextramers": usually 10 or 12 to 25 peptide-HLA-C monomers covalently attach to one dextrane molecule. The distribution width and/or maximum can depend on the type of synthesis.

In one embodiment, the multimer complex of the present invention is a HLA-C*06:02 multimer, more preferably a HLA-C*06:02 tetramer.

In one embodiment, the multimer complex of the present invention is a HLA-C*06:02 multimer, more preferably a HLA-C*06:02 dextramer,
preferably comprising 10 to 25 HLA-C monomers,
or preferably comprising 2 to 10 HLA-C monomers.

In one embodiment, the oligomerization or multimerization can be obtained by surface coating and/or the use of polymers. For example, ELISA plates or microtiter plates or Elisaspot plates can be surface-coated with peptide-HLA-C complexes by suitably coating the surfaces with dextrane or streptavidin/avidin or the like.

In one embodiment, the at least one peptide (a) of the monomer or multimer complex is altered for modifying the binding affinity of the peptide or the peptide-HLA-C complex to the TCR.

Such "altered" peptide refers to an analogue peptide with altered primary and/or secondary structure, obtained e.g. by one or more amino acid substitutions at any residue in the amino acid sequence of the peptide.
Such alteration or modification can be used to tune or modify the affinity of the peptide-protein complex (peptide/HLA-C) to the (pathogenic) T cell receptor, such as to obtain stronger agonists or antagonists to put the T cells to rest or to induce anergy or apoptosis. See Tan et al. (2015) which show that TCR binding affinity governs the functional profile of cancer-specific CD8+ T cells.
See Cole et al. (2014) which show that TCR-peptide specificity overrides affinity-enhancing TCR-MHC complex interactions.

### Pharmaceutical compositions

As discussed above, the present invention provides pharmaceutical compositions.

A pharmaceutical composition of the present invention comprises:
(i) at least one peptide of the present invention and/or a HLA-C multimer as defined herein;
   or a peptide-HLA-C complex of the present invention;
(ii) optionally, a pharmaceutically acceptable carrier and/or excipient.

In one embodiment, the pharmaceutical composition comprises two, three, four or more peptides of the present invention.

### Uses as Psoriasis biomarker

As discussed above, the present invention provides the use of
a peptide of the present invention,
a peptide-HLA-C monomer complex of the present invention,
a peptide-HLA-C multimer complex of the present invention,
a pharmaceutical composition of the present invention, or
a protein comprising or consisting of an amino acid sequence of SEQ ID NOs. 22 to 31
as biomarker for Psoriasis.

The proteins with amino acid sequence of SEQ ID NOs. 22 to 31 refer to:
ASH1L_HUMAN; **Histone-lysine N-methyltransferase ASH1L** (EC 2.1.1.43) (ASH1-like protein) (huASH1) (Absent small and homeotic disks protein 1 homolog) (Lysine N-methyltransferase 2H) and isoforms [SEQ ID NO. 22]
ATL5_HUMAN; **ADAMTS-like protein 5 (ADAMTSL-5)** (Thrombospondin type-1 domain-containing protein 6) [SEQ ID NO. 23]
HECAM_HUMAN; **Hepatocyte cell adhesion molecule** (Protein hepaCAM) [SEQ ID NO. 24]
C2C4A_HUMAN und C2C4B_HUMAN; C2 **calcium-dependent domain-containing protein 4A/4B;** Nuclear-localized factor 1 (Protein FAM148A) und Nuclear-localized factor 2) (Protein FAM148B)
**C2C4A_HUMAN** [SEQ ID NO. 25]
**C2C4B**_**HUMAN** [SEQ ID NO. 26]
THEM6_HUMAN; **Protein THEM6** (Mesenchymal stem cell protein DSCD75) (Thioesterase superfamily member 6) [SEQ ID NO. 27]
RASFA_HUMAN; **Ras association domain-containing protein 10** (RASSF10) [SEQ ID NO. 28]
Q2YHE6_HUMAN; **IL-24 splice variant deIE3** [SEQ ID NO. 29]
Q9P0E8_HUMAN; **HSPC088** [SEQ ID NO. 30]
**Hypothetical protein LOC283951, Sequence ID: gb|AAK61280.1|AE006467_6** [SEQ ID NO.31]

See also Table 2 below.

The use according the present invention comprises at least one of the following:
- monitoring disease activity;
- monitoring efficacy of treatment;
- determining disease risk; and/or
- determining the frequency of autoreactive T cells in samples of subjects having Psoriasis, preferably Psoriasis vulgaris, more preferably Psoriasis vulgaris with all types of (clinical) manifestations, such as but not limited to chronic plaque psoriasis, acute guttate psoriasis, localized or generalized pustular psoriasis, psoriatic arthritis, psoriatic nail disease and others.

"Psoriasis vulgaris" as used herein refers to Psoriasis vulgaris with all types of (clinical) manifestations, such as but not limited to chronic plaque psoriasis, acute guttate psoriasis, localized or generalized pustular psoriasis, psoriatic arthritis, psoriatic nail disease and others.

### Medical uses

As discussed above, the present invention provides
the peptide of the present invention,
the peptide- HLA-C monomer complex of the present invention,
the peptide- HLA-C multimer complex of the present invention, or
the pharmaceutical composition of the present invention,
for use in medicine.

As discussed above, the present invention provides
the peptide of the present invention,
the peptide- HLA-C monomer complex of the present invention,
the peptide- HLA-C multimer complex of the present invention,
the pharmaceutical composition of the present invention, or
a protein comprising or consisting of an amino acid sequence of SEQ ID NOs. 22 to 31
for use in the diagnosis, prevention, monitoring and/or treatment of Psoriasis, preferably Psoriasis vulgaris.

In one embodiment comprises the monitoring of Psoriasis, preferably Psoriasis vulgaris, monitoring the treatment or therapy of Psoriasis, preferably Psoriasis vulgaris.

In one embodiment,
determining the frequency of autoreactive T cells in samples of subjects having Psoriasis, preferably Psoriasis vulgaris, is comprised.

One embodiment comprises the use of a peptide of the present invention or pharmaceutical composition (comprising the peptide) of the present invention or protein of the present invention, wherein the prevention and/or treatment of Psoriasis, preferably Psoriasis vulgaris, comprises
- inhibiting or blocking the interaction of TCR and HLA-C, and/or
- suppressing the expression of HLA-C.

One embodiment comprises the use of a complex of the present invention, wherein the prevention and/or treatment of Psoriasis, preferably Psoriasis vulgaris, comprises
creating immunotolerance against the autoantigens;
affecting, tolerating or blocking the activation of autoreactive T cells for therapeutic purposes;
eliminating the pathogenic CD8⁺ T cells; and/or
down-regulating the pathogenic immune response by specifically blocking HLA-C/T-cell-interactions.

Preferably, the peptide and/or the HLA-C of the complex comprises at least a toxin or radionuclide or fluorescent dye or any other traceable marker, as described for example herein, as further component(s).

Preferably, the administration is systemic, intravenous, and/or via intradermal, subcutaneous, intramuscular, intravenous, intraosseous, intraperitoneal, intrathecal, epidural, intracardiac, intraarticular, intracavernous, intracerebral, intracerebroventricular and intravitreal injection(s).

### Methods of treatment

As discussed above, the present invention provides a method for the diagnosis, prevention, monitoring and/or treatment of Psoriasis, preferably Psoriasis vulgaris.

Said method comprises the step of
administering a therapeutically effective amount of
at least one peptide of the present invention,
a peptide- HLA-C monomer complex of the present invention,
a peptide- HLA-C multimer complex of the present invention,
a pharmaceutical composition of the present invention, or
a protein comprising or consisting of an amino acid sequence of SEQ ID NOs. 22 to 31
to a subject in need thereof.

Preferably, the administration is systemic, intravenous, and/or via intradermal, subcutaneous, intramuscular, intravenous, intraosseous, intraperitoneal, intrathecal, epidural, intracardiac, intraarticular, intracavernous, intracerebral, intracerebroventricular and intravitreal injection(s).

In one embodiment, the peptide and/or the HLA-C of the complex comprises at least a toxin or radionuclide or fluorescent dye or any other traceable marker, as described for example herein, as further component(s).

### Means and methods for inhibiting or blocking the interaction of TCR and HLA-C

As discussed above, the present invention provides a compound that is directed against HLA-C for use in the prevention, and/or treatment of Psoriasis, preferably Psoriasis vulgaris.

Said compound is selected from an anti-HLA-C antibody or fragment thereof (such as Fab, Fv, scFv), a small molecule (inhibitor), a small interfering RNA.

Preferably the prevention and/or treatment of Psoriasis, preferably Psoriasis vulgaris, comprises
(a) inhibiting or blocking the interaction of TCR and HLA-C, and/or
(b) suppressing the expression of HLA-C.

As discussed above, the present invention provides a method for the prevention and/or treatment of Psoriasis, preferably Psoriasis vulgaris, comprising
(a) inhibiting or blocking the interaction of TCR and HLA-C, and/or
(b) suppressing the expression of HLA-C.

In one embodiment, the inhibiting or blocking the interaction of TCR and HLA-C (a) comprises: interfering with the contact between TCR and HLA-C
such as but not limited to antibodies (monoclonal antibodies, antibody fragments, e.g. Fab, Fv, scFv) or other molecules directed specifically against HLA-C.

In one embodiment, the suppressing the expression of HLA-C (b) comprises:
suppressing the expression of HLA-C on cell surfaces by means
   such as but not limited to small interfering RNAs or other small molecules that are designed to reduce HLA-C transcription, HLA-C translation or HLA-C transport to the cell surface.

### Further description of preferred embodiments

### - Abstract

Proteins Histone-lysine N-methyltransferase ASH1L, ADAMTS-like protein 5 (ADMTSL5), Ras association domain-containing protein 10 (RASSF10), Hepatocyte cell adhesion molecule (HEPACAM), Protein THEM6, C2 calcium-dependent domain-containing protein 4A/4B (C2CD4A/4B), IL-24 splice variant delE3, HSPC088 and hypothetical protein LOC283951 and/or peptides from said proteins and/or corresponding mimotopes for the diagnosis and treatment of psoriasis either alone or in the complex with HLA-C*06-monomers or multimers.
Blocking the interaction between T-cell receptor (TCR) and Human Leukocyte Antigen (HLA)-C molecules or any mechanism that downregulates HLA-C in skin or in other tissues as a specific treatment for psoriasis vulgaris.

The present invention relates to selected autoantigens, corresponding peptide epitopes and altered peptide ligands, such as corresponding mimotopes, as diagnostic tools or therapeutic agents, compositions comprising them as well as processes for the diagnosis, monitoring and treatment of psoriasis vulgaris.

The present invention also relates to the application of HLA-C-directed approaches that block the interaction with T-cell antigen receptors and thus inhibit T-cell activation for the specific treatment of psoriasis vulgaris in psoriasis patients.

The present invention is based on the discovery that the human leukocyte antigen HLA-C*06:02 directs an autoimmune response against melanocytes and presents autoantigens to pathogenic T cells in psoriasis. Using a recombinant TCR from a pathogenic lesional psoriatic T-cell clone of a HLA-C*06-positive psoriasis patient it identifies epitopes from autoantigenic proteins in psoriasis. It shows that patients have circulating T cells in peripheral blood, which specifically react against complexes composed of these peptides and HLA-C*06, and that complexes of said peptides and HLA-C*06 can be used to identify these peptide-specific T cells. The invention also shows that blocking the interaction between HLA-C*06 and a pathogenic T-cell antigen receptor can inhibit T-cell activation. Blocking the interaction between HLA-C molecules and TCR is therefore a treatment modality in psoriasis.

Psoriasis vulgaris is a frequent HLA-C*06:02-associated T-cell mediated autoimmune skin disease. The invention-related findings are based on the pioneering usage of a human TCR to
- determine the functional role of HLA-C*06:02 in presenting autoantigenic peptides,
- identify the target cell of the skin-specific autoimmune response,
- identify specific antigens of the autoimmune response.

HLA-C*06:02 is the main psoriasis risk allele and present in 2/3 of all patients with psoriasis.

Herein we prove for the first time,
- that HLA-C*06:02 acts by directing a T-cell mediated autoimmune response of CD8⁺ T cells against melanocytes,
- that HLA-C*06:02 presents autoantigenic peptides to pathogenic CD8⁺ T cells,
- identify said antigenic peptides peptides that are presented as autoantigens from melanocytes
- show, that blocking the interaction between the HLA-C*06:02/peptide complex with a T-cell receptor from CD8⁺ T cells inhibits antigen-specific immune activation.

The invention can be used for:
(i) Diagnosis of psoriasis:
   - Use of multimeric peptide/HLA-C*06:02 complexes as biomarker for monitoring disease activity,
      monitoring efficacy of treatment,
      defining disease risk.
(ii) Treatment of psoriasis:
   - Use of altered peptide ligands for creating immunotolerance against the autoantigens,
   - Provide a cure by eliminating the pathogenic CD8⁺ T cells using toxin-coupled multimeric peptide/HLA-C*06:02 complexes,
   - Downregulate the pathogenic immune response by specifically blocking HLA-C/T-cell-interactions.

### - Results

### Generation of Vα3S1/Vβ13S1 TCR hybridoma

We had previously developed a technique for the molecular characterization of the paired TCR α and β-chain rearrangements of single T cells. Herewith, we were able to prove that the majority of CD8⁺ T cells, which infiltrate into the epidermis of psoriatic plaques, are clonally expanded (Kim et al., 2012). Clonal T-cell expansions in inflammatory tissue lesions are indicative of antigen-driven T-cell responses and characterize presumably pathogenic T cells (Kent et al., 2005; see also WO 2012/017081 A1).

Now, we cloned the cDNA of the α- and β-chains of a functional Vα3S1/Vβ13S1 TCR from a predominant epidermal psoriatic CD8⁺ T-cell clone of an HLA-C*06:02-positive psoriasis patient. Evidence of psoriasis-related pathogenicity of the donor T cells is based on three observations:
(1) Multiple dermal and epidermal CD8⁺ T cells from a lesional psoriasis biopsy carried the same TCR α-chain (Vα3S1-NN-Jα 45.1: CA TDAL YSGG) and β-chain rearrangements (Vβ13S1-N(D)N-Jβ 1.1: CASSY SEGED EAFF; Arden nomenclature (Arden et al., 1995), deduced amino acid sequence, one letter amino acid code).
(2) The clonal Vα3S1/Vβ13S1 TCR was also identified in a second, independently examined biopsy part from the same patient (Kim et al., 2012).
(3) CD8⁺ T cells in psoriatic epidermis have been described to preferentially rearrange Vβ13S1 (Chang et al., 1997).

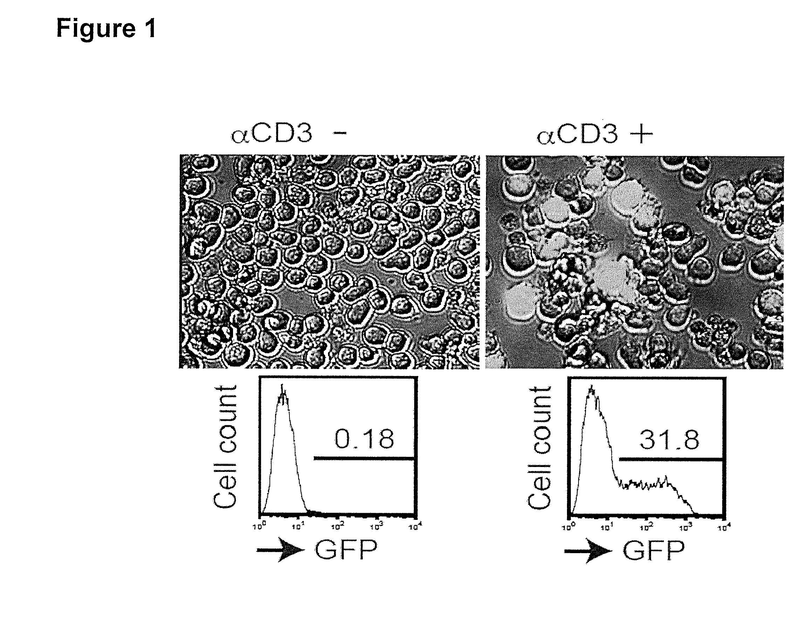

We co-expressed this Vα3S1/Vβ13S1 TCR together with human CD8 α and β chains and super green fluorescence protein (sGFP) under the control of nuclear factor of activated T cells (NFAT) in the TCR-deficient mouse reporter T-hybridoma cell line 58α⁻β⁻ (Seitz et al., 2006). Upon TCR ligation, Vα3S1/Vβ13S1 TCR hybridoma cells are activated and produce sGFP, which can be detected by ultraviolet (UV) fluorescence microscopy or fluorescence activated cell sorting (Figure 1). When assessing the results given below, however, it needs to be considered that the percentage of activated i.e. green-fluorescing cells in this complex cell-based recombinant assay may differ between different experiments. It may be influenced by the stability of the respective hybridoma cell batch and, in co-culture experiments, by the quantitative ratio of stimulatory and hybridoma cells, which decides the probability of cell-cell contacts (Siewert et al., 2011).

*The Vα3S1*/*Vβ13S1 reacts against melanocytes in an HLA-C*06:02 - restricted manner* Given that the Vα3S1/Vβ13S1-TCR hybridoma carries the antigen-specificity of pathogenic psoriatic CD8⁺ T cells, we used it to determine HLA-class I restriction and antigen-specificity of the lesional psoriatic immune response. The TCR origin from a lesional epidermal CD8⁺ T-cell clone of an HLA-C*06:02-positive psoriasis patient indicated an HLA-C*06:02-restricted autoreactivity against skin-resident tissue cells. Therefore, we assessed the effect of various skin-derived cell types on the activation of the Vα3S1/Vβ13S1-TCR hybridoma in co-culture experiments.

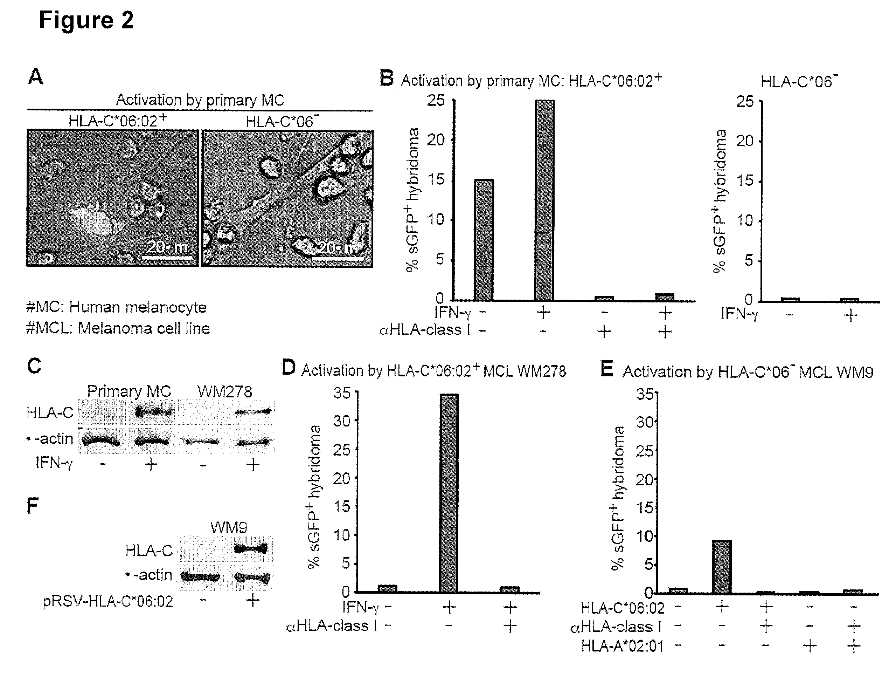

We isolated primary melanocytes from the skin and seeded them in explant culture. Cell type was confirmed by the contents of brownish melanosomes and staining with a p75-specific antibody against melanocytes. The Vα3S1/Vβ13S1-TCR hybridoma cells were activated upon co-culture with primary human melanocytes derived from the skin of HLA-C*06:02-positive but not HLA-C*06:02-negative donors, as evidenced by the induction of sGFP (Figures 2A and 2B). This effect was further enhanced, when HLA-C*06:02-positive melanocytes were preincubated with interferon (IFN)-γ, which increases the expression of HLA-class I molecules (Figure 2C). The stimulatory capacity was observed in HLA-C*06:02-positive melanocytes obtained from both skin of psoriasis patients or healthy donors, indicating a genuine antigenicity of melanocytes in the context of HLA-C*06:02. TCR-hybridoma activation was HLA-restricted. It was increased by the addition of IFN-γ, which enhances the expression of HLA-C, and completely blocked by a pan HLA-class I antibody, W6/32, that inhibits TCR-HLA interactions (Figure 2B).

Consistent results were obtained with two inherently HLA-C*06:02-positive melanocytic cell lines, 1205LU and WM278: cells from both cell lines strongly activated the TCR hybridoma when preincubated with IFN-γ to induce the expression of HLA-C (Figure 2D). Again, this effect was reverted by a blocking pan-HLA-class I antibody, W6/32. The HLA-C*06:02-negative melanocytic cell lines, WM9, WM1232 or WM239, activated Vα3S1/Vβ13S1 TCR hybridoma cells in co-culture experiments only after transfection of HLA-C*06:02, but not HLA-A*0201 (Figure 2E). The stimulatory capacity of these HLA-C*06:02-transfected cell lines was independent from addition of IFN-γ.

No hybridoma activation was observed in co-culture experiments with HLA-C*06:02-transfected primary human keratinocytes, cells from the epidermal cell line, HaCaT (Boukamp et al., 1988) or A431, nor with HLA-C*06:02-positive primary skin fibroblasts (*data not shown*), indicating a specific role of melanocytes in TCR activation. Thus, our results imply a selective reactivity of the psoriatic Vα3S1/Vβ13S1-TCR of a presumably pathogenic T-cell clone against self-antigens presented by melanocytes in dependence of HLA-C*06:02-surface expression.
→ HLA-C directs an autoimmune response against melanocytes in psoriasis. Increasing the expression of HLA-C enhances TCR-mediated T-cell activation. Vice versa, blocking the interaction between HLA-C and TCR blocks TCR-mediated T-cell activation. Blocking the interaction between HLA-C and TCR is a relevant approach for the treatment of psoriasis.
→ Given the role of HLA-C in presenting the autoantigens any mechanism that downregulates the expression of HLA-C in skin lesions or other tissues can be used to control the autoimmune response and be employed for treatment of psoriasis.

*The Vα3S1*/*Vβ13S1 TCR reacts against different peptides with a convergent amino acid pattern that facilitates the identification of stimulatory natural human peptide homologues* To characterize potential melanocytic self-antigens, we searched for peptide motifs that are recognized by the Vα3S1/Vβ13S1 TCR in the context of HLA-C*06:02. For this purpose, we used a randomized plasmid peptide library, encoding nonameric peptides (Siewert et al., 2011). In previous experiments we had shown that this approach is suitable for identifying HLA-restricted peptide antigens of a TCR specific for a defined influenza virus antigen. COS-7 cells were cotransfected with the peptide library and pRSV-HLA-C*06:02 and incubated with Vα3S1/Vβ13S1-TCR hybridoma cells. COS-7 cells adjacent to activated TCR hybridoma cells were picked and subjected to plasmid subcloning and sequencing to identify the encoded antigenic peptide as described (Siewert et al., 2011).

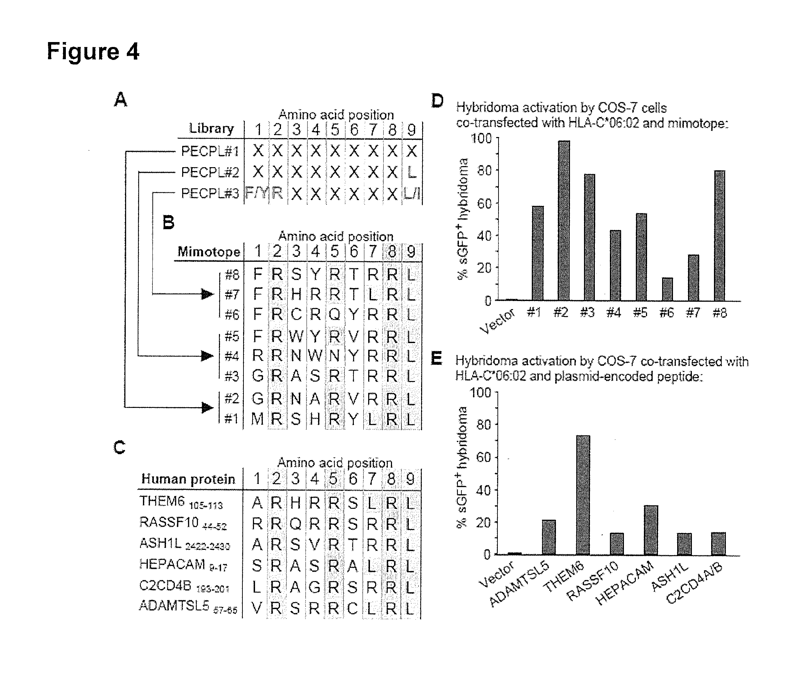

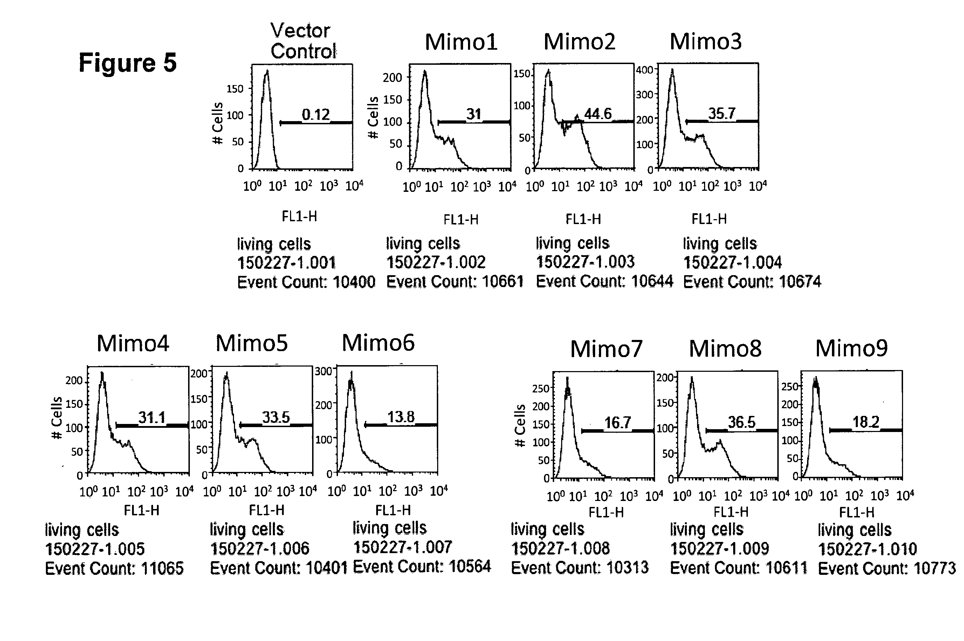

We identified nine different nonamer mimotopes, i.e. artificial peptide epitopes that ligated the Vα3S1/Vβ13S1 TCR in the context of HLA-C*06:02 (Table 1, Figure 4 and 5).

**Table 1. Amino acid sequence of mimotopes activation Vα3S1/Vβ13S1 TCR hybridoma cells**

| **Mimotope** | Amino acid positions P1-P9 | | | | | | | | |
|---|---|---|---|---|---|---|---|---|---|
| SEQ ID NO. | ***P1*** | **P2** | ***P3*** | ***P4*** | **P5** | ***P6*** | **P7** | **P8** | **P9** |
| 1 | *F* | **R** | *S* | *Y* | **R** | *T* | R | **R** | **L** |
| 2 | *F* | **R** | *H* | *R* | **R** | *T* | L | **R** | **L** |
| 3 | *F* | **R** | *C* | *R* | **Q** | *Y* | R | **R** | **L** |
| 4 | *F* | **R** | *W* | *Y* | **R** | *V* | R | **R** | **L** |
| 5 | *R* | **R** | *N* | *W* | **N** | *Y* | R | **R** | **L** |
| 6 | *G* | **R** | *A* | *S* | **R** | *T* | R | **R** | **L** |
| 7 | *G* | **R** | *N* | *A* | **R** | *V* | R | **R** | **L** |
| 8 | *M* | **R** | *S* | *H* | **R** | *Y* | L | **R** | **L** |
| 9 | *R* | **R** | *Y* | *F* | **R** | *C* | L | **R** | **M** |

| | | | | | | | | | |
|---|---|---|---|---|---|---|---|---|---|
| Amino acids are given as single letter amino acid code **Bold: conserved amino acid pattern in positions P2, P5, P8 and P9;** *italic: diversity in positions P1, P3, P4 and P6.* | | | | | | | | | |

These nonamers exhibited diversity in positions P1, P3, P4 and P6. However, our analysis revealed a conserved amino acid pattern in positions P2 and P8 (Arg), P9 (Leu or Met) and P5 (Arg, Glu or Asn), indicating a high relevance of these amino acids for the interaction with the Vα3S1/Vβ13S1 TCR. The observed polyspecificity of the Vα3S1/Vβ13S1 TCR is consistent with the recognition behaviour of TCRs reported previously (Wucherpfennig et al., 2007). Defined TCRs may cross-react with many distinct peptides, which, however, share conserved TCR-binding motifs that can be used to identify the naturally occurring TCR ligands ((Siewert et al., 2011; Birnbaum et al., 2014).

To identify the corresponding natural psoriatic antigen of the Vα3S1/Vβ13S1 TCR, we screened the mimotopes against the non-redundant human protein database and the transcriptome of 1205LU cells. However, we were unable to identify proteins containing peptides that were identical to any of the mimotopes in this way. For further homology searches, we therefore generated various algorithms based on the converging amino acid pattern of the above mimotopes. This allowed us to select 185 different peptides from natural human proteins for further analysis.

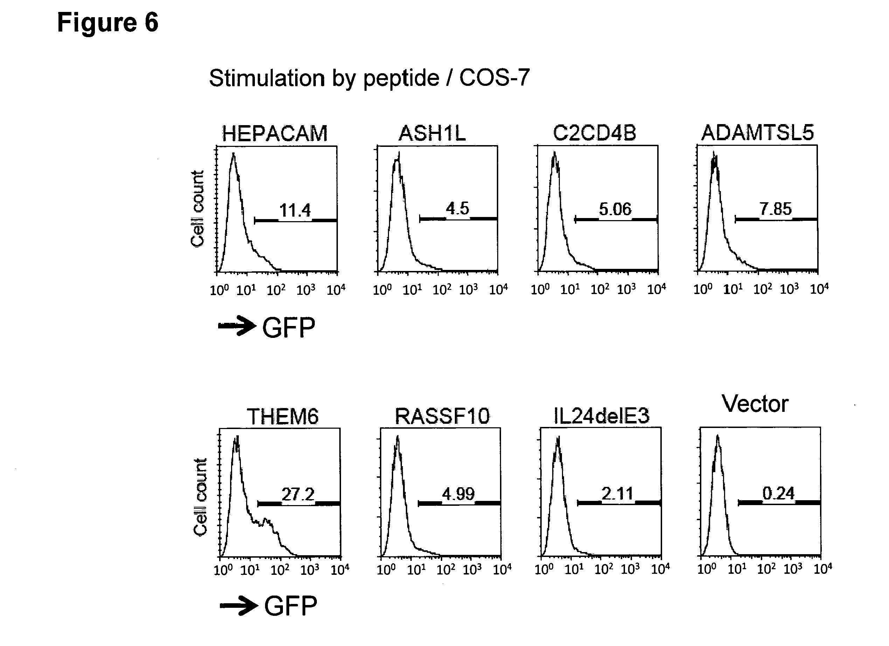

After cloning into an expression vector they were co-transfected with HLA-C*06:02 into COS-7 cells and tested for their ability to activate Vα3S1/Vβ13S1 TCR hybridoma cells in co-culture experiments. Nine of these peptide epitopes stimulated the Vα3S1/Vβ13S1 TCR hybridoma (Table 2; Figure 6: Antigen-specific activation of Vα3S1/Vβ13S1 TCR hybridoma by select peptides). Thus, analysis of the convergent amino acids pattern of library mimotopes allowed for the identification of several putative self-epitopes presented by HLA-C*06:02 and recognized by the Vα3S1/Vβ13S1 TCR.

**Table 2. Peptides from natural human proteins that activate the Vα3S1/Vβ13S1 TCR hybridoma**

| **#** | **UniProt Accession #** | **Uniprot entry** | **Protein name** | **Gen name** | Peptide sequence |
|---|---|---|---|---|---|
| 1 | Q9NR48 | ASH1L_ HUMAN | **Histone-lysine N-methyltransferase ASH1L** (EC 2.1.1.43) (ASH1-like protein) (huASH1) (Absent small and homeotic disks protein 1 homolog) (Lysine N-methyltransferase 2H) and isoforms | **ASH1L,** KIAA1420, KMT2H | ARSVRTRRL SEQ ID NO. 13 |
| 2 | Q6ZMM2 | ATL5_ HUMAN | **ADAMTS-like protein 5** (ADAMTSL-5) (Thrombospondin type-1 domain-containing protein 6) | **ADAMTSL5,** THSD6 | VRSRRCLRL SEQ ID NO. 17 |
| 3 | Q14CZ8 | HECAM_ HUMAN | **Hepatocyte cell adhesion molecule** (Protein hepaCAM) | **HEPACAM** | SRASRALRL SEQ ID NO. 14 |
| 4 | Q8NCU7 A6NLJ0 | C2C4A_ HUMAN C2C4B_ HUMAN | **C2 calcium-dependent domain-containing protein 4A**/**4B** Nuclear-localized factor 1 (Protein FAM148A) Nuclear-localized factor 2) (Protein FAM148B) | **C2CD4A/B,** FAM148B, NLF2 | LRAGRSRRL SEQ ID NO. 15 |
| 5 | Q8WUY1 | THEM6_ HUMAN | **Protein THEM6** (Mesenchymal stem cell protein DSCD75) (Thioesterase superfamily member 6) | **THEM6,** C8orf55, PSEC0098 | ARHRRSLRL SEQ ID NO. 10 |
| 6 | A6NK89 | RASFA_ HUMAN | **Ras association domain-containing protein 10** | **RASSF10** | RRQRRSRRL SEQ ID NO. 11 |
| 7 | Q2YHE6 | Q2YHE6_ HUMAN | **IL-24 splice variant deIE3** | **N/A** | RRFCRTSRM SEQ ID NO. 19 |
| 8 | Q9P0E8 | Q9P0E8_H UMAN | **HSPC088** | | HRHSRILRL SEQ ID NO. 20 |
| 9 | | GenBank: AAK6128 0.1 | **hypothetical protein LOC283951, isoform CRA_**a | | SRAVRALRL SEQ ID NO. 21 |

→ Peptides from several natural human proteins constitute autoantigens for psoriasis when complexed with HLA-C*06:02 and activate a representative pathogenic psoriatic TCR. Mimotopes (artificial TCR peptide ligands) sharing a particular amino acid pattern can be used to replace these natural peptide ligands for TCR activation. Due to the polyspecificity/crossreactivity of TCRs, a single TCR may recognize all these different natural peptides. Both mimotopes and natural peptide ligands can therefore be used to address pathogenic psoriatic T cells in psoriasis in the context of HLA-C*06 for diagnostic or therapeutic purposes.

### Staining of PBMC from Psoriasis patients with HLA-C*06:02-peptide dextramers

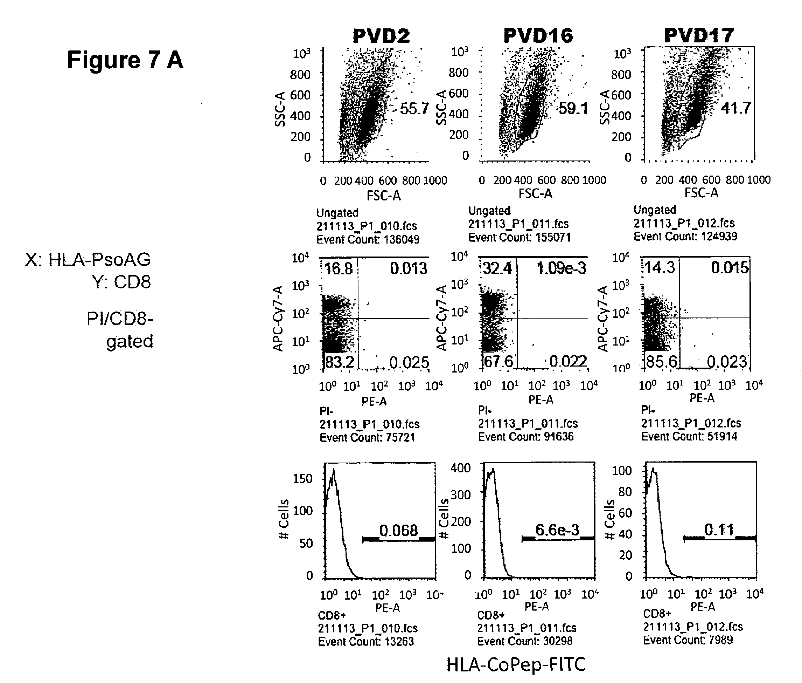

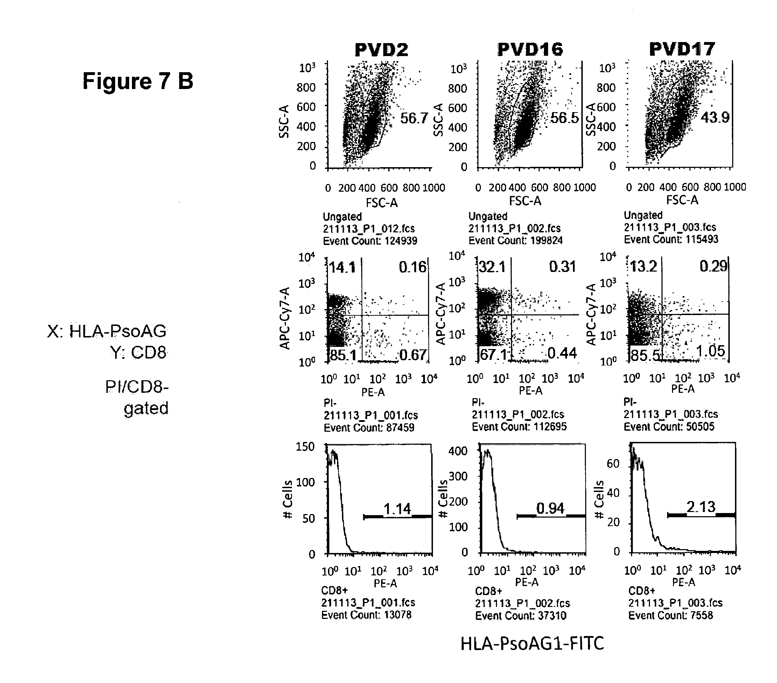

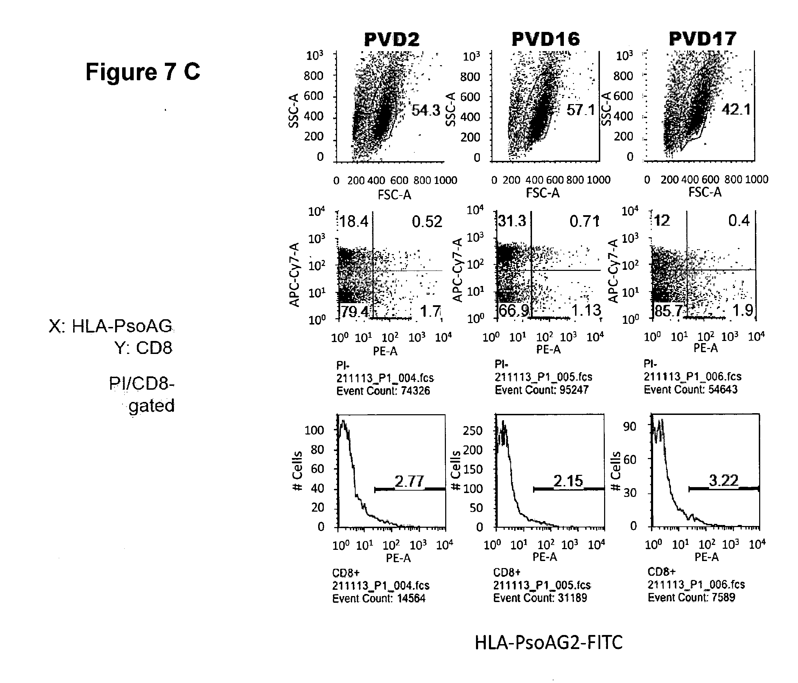

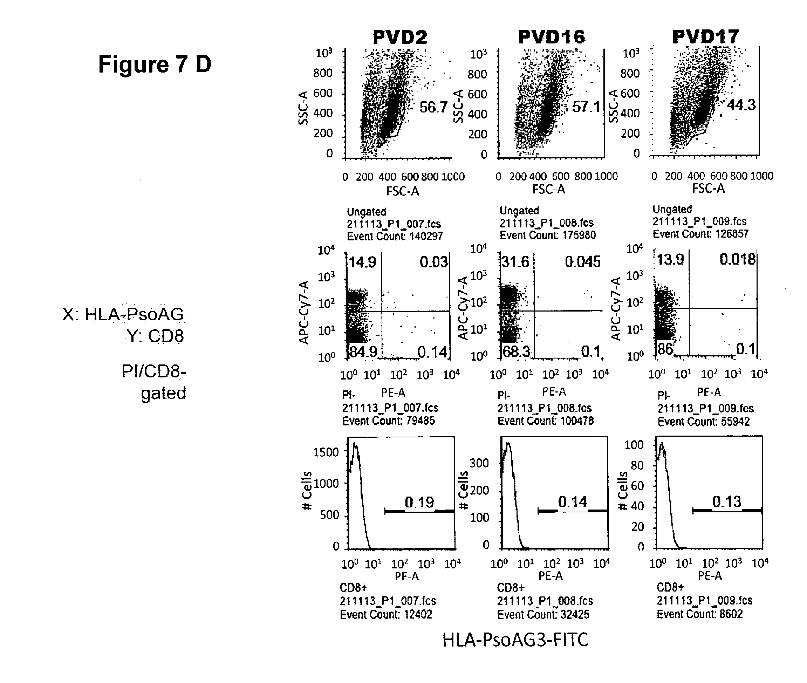

We used FITC-labelled HLA-C*06:02-peptide dextramers to stain CD8⁺ T cells from psoriasis patients. Only HLA-C*06:02-dextramers containing antigenic peptides for the Vα3S1/Vβ13S1 TCR stained CD8⁺ T cells. As negative control we used an unrelated peptide, VRHDGGNVL (Falk et al., 1993), complexed with HLA-C*06:02-dextramers did not bind to blood T cells from psoriasis patients (Figure 7). Thus, the peptides can be used in complex with HLA-C dextramers to address autoreactive T cells in blood of psoriasis patients.
→ Autoantigenic peptides complexed with HLA-C*06:02-multimers can be used to identify autoreactive CD8⁺ T cells in psoriasis. This mechanism can be used as biomarker to measure the frequency of autoreactive T cells from biologic samples (blood, skin, lymph nodes etc) in order to support the diagnosis of psoriasis or to monitor the course of disease or assess the risk for disease onset in individuals with a genetic predisposition for psoriasis.
→ Autoantigenic peptides complexed with toxin-conjugated HLA-C*06:02- dextramers can be used for an antigen-specific elimination of autoreactive pathogenic CD8⁺ T cells in psoriasis.
→ Altered peptide ligands complexed with HLA-C*06:02-dextramers that bind the pathogenic TCRs can be used to affect or tolerize or block the activation of autoreactive T cells for therapeutic purposes.
→ Peptides alone or together can be used to measure the T-cell responsiveness in stimulation assays in order to determine specific immune responses in patients.

The following examples and drawings illustrate the present invention without, however, limiting the same thereto.

### BRIEF DESCRIPTION OF THE DRAWINGS

**Figure 1****.** *Vα3S1*/*Vβ13S1-TCR hybridoma activation by CD3-antibody.*
   Shown is activation of hybridoma cells by a CD3 monoclonal antobody, that leads to the production of green fluorescent protein. This can be visualized by fluorescence microscopy or determined by FACS analysis.
**Figure 2****.** *HLA-C*06:02-Restricted Activation of the CD8⁺ Vα3S1*/*Vβ13S1 TCR Reporter Hybridoma Cell Line by Melanocytes.*
   (A) TCR-hybridoma activation by primary human melanocytes (MC, elongated cells). Merged light and fluorescence microscopy images of Vα3S1/Vβ13S1-TCR hybridoma cells (round shapes) that were activated to express sGFP upon co-culture with HLA-C*06:02-positive (left) but not HLA-C*06:02-negative melanocytes (right).
   (B) TCR-hybridoma activation by HLA-C*06:02-positive (left) or HLA-C*06:02-negative (right) primary human melanocytes (MC) in the absence or presence of IFN-γ or a HLA-class I blocking antibody. Frequency of NFAT-sGFP⁺ hybridoma cells was assessed by flow cytometry and normalized to the maximum value induced by CD3 activation in the same experiment. Data are representative of three HLA-C*06:02-positive (healthy n=2, psoriasis n=1) and nine HLA-C*06:02-negative individuals (healthy n=7, psoriasis n=2).
   (C) Western blot analysis of HLA-C (upper panel) and β-action expression (loading control, lower panel).in lysates from primary human melanocytes (MC, left) or the melanoma-derived cell line WM278 (right) cultured in the absence or presence of IFN-γ.
   (D) TCR-hybridoma activation by co-culture with the HLA-C*06:02-positive melanocytic cell line (MCL) WM278 preincubated without or with IFN-γ or a HLA-class I blocking antibody. Data were assessed as in (B).
   (E) TCR-hybridoma activation by HLA-C*06:02-negative melanocytic cell line (MCL) WM9 transfected with HLA-C*06:02 or HLA-A*0201 and incubated with an anti-HLA-class I antibody. Data were assessed as in (B). (F) Western blot analysis of HLA-C (upper panel) and β-action expression (loading control, lower panel) in lysates of WM9 cells transfected with pRSV-HLA-C*06:02.
      Data represent 3 (C), >3 (D), and 3 (E) independent experiments.

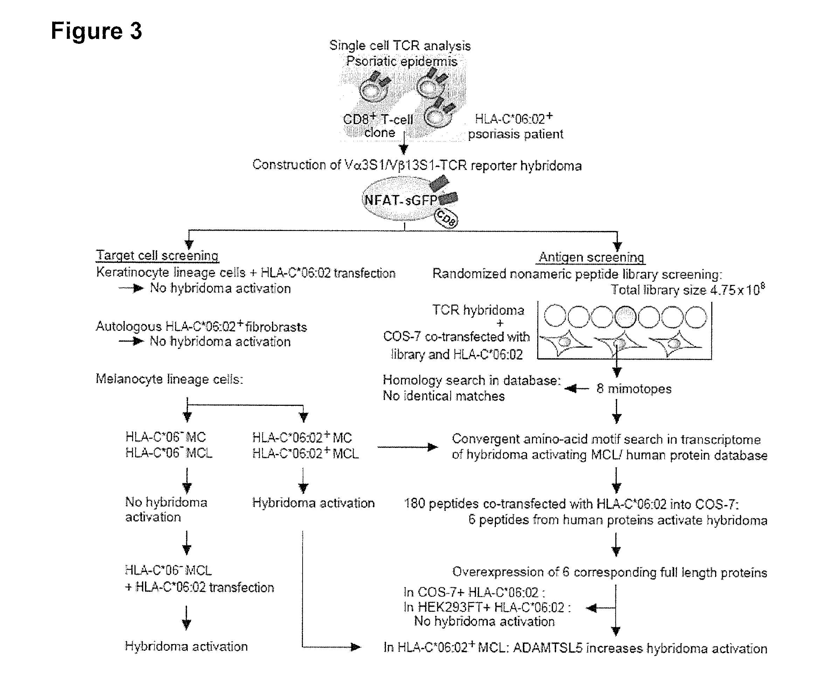

**Figure 3****.** *Screening Strategy to Determine the Vα3S1*/*Vβ13S1 TCR Reactivity.*
   Schematic of screening strategy to identify cellular targets (left) or antigens (right) of the Vα3S1/Vβ13S1-TCR. Target cell screening involved co-cultured with different skin cell types. MC, primary human melanocytes. Antigen screening by nonamer peptide libraries was followed by transfection of candidate peptide epitopes and full-length proteins into HLA-C*06:02-transfected HEK293FT or COS-7 cells, or HLA-C*06:02-positive melanoma cell lines (MCL).
**Figure 4****.** *Identification of Peptide Ligands of the Vα3S1*/*Vβ13S1 TCR.*
   (A) Design of plasmid-encoded combinatorial nonamer peptide libraries (PECPL) #1-3 with predefined amino acid residues in single letter amino acid code. X: randomized residue.
   (B) Mimotopes derived from PECPL#1-3 activating the Vα3S1/Vβ13S1-TCR hybridoma. HLA-C*06:02 anchor positions are labelled in yellow, conserved amino acids of mimotopes independent from anchor positions in green, blue or red. See also Figure S3.
   (C) TCR-hybridoma activation by mimotopes co-transfected with HLA-C*06:02 into COS-7 cells. Frequency of NFAT-sGFP⁺ hybridoma cells was assessed by flow cytometry and normalized to the maximum value induced by CD3 activation in the same experiment.
   (D) Natural human peptide ligands activating the Vα3S1/Vβ13S1-TCR hybridoma. Subscript numbers give amino acid positions in parent proteins. Labelling as in (B).
   (E) TCR-hybridoma activation by plasmid-encoded natural human peptide epitopes co-transfected with HLA-C*06:02 into COS-7 cells. Data were assessed as in (C).
**Figure 5****.** *Stimulation of Vα3S1*/*Vβ13S1 TCR hybridoma cells by mimotopes 1-9 co-transfected with HLA-C*06:02 into COS-7 cells.*
   Flow cytometry analysis of NFAT-sGFP⁺ Vα3S1/Vβ13S1 TCR hybridoma cells following co-culture with COS-7 cells co-transfected with plasmids containing mimotopes and HLA-C*06:02.
**Figure 6****.** *Stimulation of Vα3S1*/*Vβ13S1 TCR hybridoma cells by natural human peptide ligands co-transfected with HLA-C*06:02 into COS-7 cells.*
   Flow cytometry analysis of NFAT-sGFP⁺ Vα3S1/Vβ13S1 TCR hybridoma cells following co-culture with COS-7 cells co-transfected with plasmids containing natural human peptide epitopes and HLA-C*06:02.
**Figure 7****.** *Staining of peripheral blood CD8*⁺ *T cells with peptide-HLA-C*06:02 dextramers.*
   (A) HLA-C*06:02/control peptide
   (B) HLA-C*06:02/RASSF10 peptide
   (C) HLA-C*06:02/HEPACAM peptide
   (D) HLA-C*06:02/IL24del3 peptide
   Shown is the FACS analysis of CD8⁺ T cells stained with labelled peptide/HLA-C*06:02-dextramers. HLA-C*06:02/control peptide refers to an unrelated peptide formerly isolated from HLA-C*06:02.

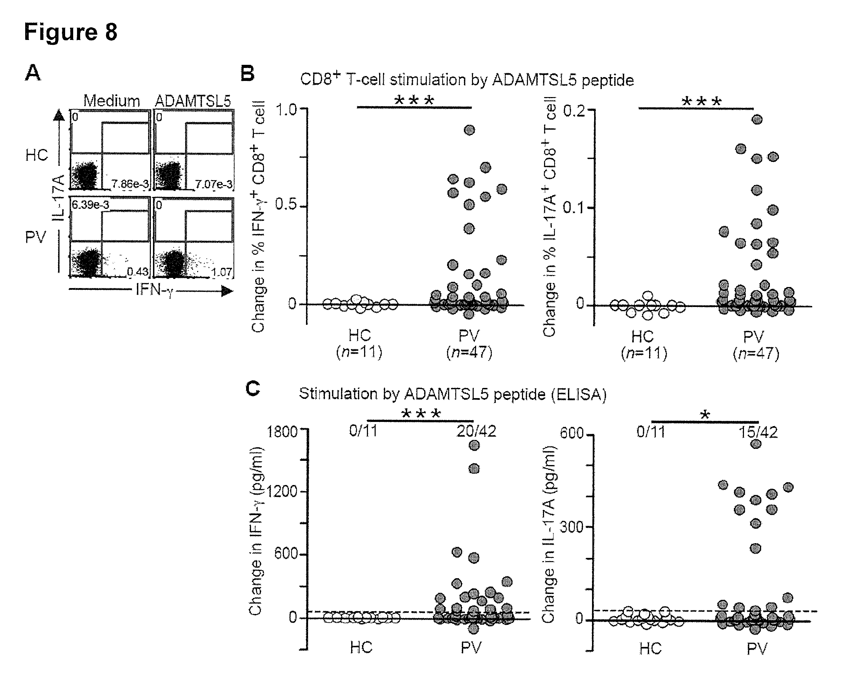

**Figure 8****.** *Cytokine Induction by Stimulation of PBMC with ADAMTSL5 Peptide.*
   (A) Intracellular staining of IFN-γ and IL-17A in CD8⁺ T cells following stimulation of PBMC with synthetic ADAMTSL5 peptide (right) or medium control (left). Representative histograms show flow cytometry analysis of each a representative experiment for a healthy control (HC) and a psoriasis patient (PV).
   (B) Changes in frequencies of IFN-γ⁺- and IL-17A⁺ CD8⁺ T cells after ADAMTSL5 peptide stimulation of PBMC from healthy controls (HC, n=11) and psoriasis patients (PV, n=47) after normalization to medium control. Each dot represents one individual. ****p*<0.005 (Mann-Whitney U test).
   (C) Changes in IFN-γ (left) and IL-17A concentrations (right) in culture supernatants of PBMC from healthy controls (HC, n=11) or psoriasis patients (PV, n=37) following stimulation with synthetic ADAMTSL5 peptide as determined in triplicates by ELISA. Graphs depict changes by ADAMTSL5 peptide over medium control, each dot represents one individual. Dashed line indicates threshold. ****p*<0.005, ***p*<0.01 (Chi square test).

### EXAMPLES

### 1. Experimental Procedures

### 1.1 Human Subjects

Lesional biopsies were obtained from patients with chronic plaque psoriasis. Blood samples were obtained by antecubital venous puncture. All participants gave written informed consent. The study was performed in accordance with the Helsinki Declaration and approved by the Ethics committee of the university hospital

### 1.2 Generation of Vα3S1/Vβ13S1-TCR CD8⁺ reporter T-hybridoma

Identification of the matching Va3S1 and Vβ13S1-TCR chains of a CD8⁺ T-cell clone (Vα3S1-NN-Jα 45.1: CA TDAL YSGG, Vβ13S1-N(D)N-Jβ 1.1: CASSY SEGED EAFF; Arden nomenclature, see Arden et al. 1995) has been described (see Kim et al. 2012). TCR-hybridoma generation was performed as described (Seitz et al., 2006; Siewert et al., 2011). Briefly, Vα and Vβ-regions were cloned into expression plasmids pRSV-hygro (α-chain) and pRSV-neo (β-chain) using restriction sites SalI-PvuII or SalI-BIpI. The resulting plasmids were linearized (XmnI and NdeI, respectively) and electroporated into 58 α⁻β⁻ T hybridoma cells. Functional clones were supertransfected with pLPCX-CD8α-IRES-β and pcDNA-NFAT-sGFP. TCR-activation induced NFAT-sGFP-expression was determined by CD3 crosslinking with anti-mouse CD3 antibody (clone 17A2, eBioscience), flow cytometry and fluorescence microscopy (AxioVert200M, Zeiss, 520/35 BrightLine filter, Semrock and 605/70 filter). Clones with highest frequencies of responding cells (usually ≥30 % NFAT-sGFP-positive cells after CD3 stimulation) were expanded in T-hybridoma medium (see "Primary cells and cell lines" section). Hybridoma batches were frequently recloned to minimize sporadic sGFP expression and decrease of activation rates. The resulting cell line is termed Vα3S1/Vβ13S1-TCR hybridoma.

### 1.3 Construction of plasmid-encoded combinatorial peptide libraries (PECPL) and identification of Vα3S1/Vβ13S1-TCR mimotopes

Completely randomized nonameric PECP library #1, PECPL #2 with predetermined amino acids Leu at P9 or #3 with Phe/Tyr at P1, Arg at P2 and Leu/Ile at P9 were prepared as described⁴. Screening strategy and library designs and are shown in Fig. 3 and Fig. 4. COS-7 cells were co-transfected with PECP-libraries and HLA-C*06:02, co-cultured with Vα3S1/Vβ13S1-TCR hybridoma and screened by UV-microscopy for the induction of sGFP. Mimotope-containing plasmids were isolated and sequenced.

PECPL screening was performed as described (Siewert et al., 2011). COS-7 cells were cotransfected with PECPLs and HLA-C*06:02 and cocultured with Vα3S1/Vβ13S1-TCR hybridoma cells. After 16 h, COS-7 cells in close contact to fluorescent hybridoma cells were isolated and library peptides were amplified by nested PCR. PCR products were cloned into pcDNA.3.1D/V5-His-TOPO and transformed into *E.coli.* The mimotope-enriched library plasmids were cotransfected with pRSV-HLA-C*06:02 into COS-7 cells and the mimotope-containing plasmid was isolated by subcloning and sequencing.

Amino acid sequences of mimotopes were aligned and used in different search strategies to identify corresponding peptides from human proteins in the taxa-specific *homo sapiens* [9606] UniProt database or melanocytic transcriptome. cDNA corresponding to 180 candidate peptide antigens or six corresponding full-length proteins were cloned into pcDNA3.1D/V5-His-TOPO. Ectopic protein expression was verified by Western blotting. Mutations of ADAMTSL5 epitope₅₈₋₆₅ were introduced by PCR using internal reverse primers. siRNAs were transfected into WM278 cells for ADAMTSL5 knock down. Knock down efficacy was examined by triplicate quantitative PCR using PBGD as an internal standard.

### 1.4 Cells, cell lines and Vα3S1/Vβ13S1-TCR hybridoma activation assays

Primary human melanocytes were cultured from skin samples (Hsu and Herlyn, 1996). Peripheral blood mononuclear cells were prepared by density gradient centrifugation. Human neonatal epidermal keratinocytes were obtained commercially. The following cell lines were used: COS-7, HEK293FT, HaCaT, A431, WM278, WM9, WM239A, 1205Lu.

Activation of Vα3S1/Vβ13S1-TCR hybridoma cells was assessed in co-culture experiments by NFAT-sGFP induction and determined by flow cytometry and UV-fluorescence microscopy. IFN-γ was added to increase HLA-class I expression of HLA-C*06:02-positive cells/cell lines. HLA-C*06:02-negative cells or cell lines were transfected with pRSV-HLA-C*06:02 or pRSV-HLA-A*0201. Cell lines were transfected with plasmid-encoded peptides or full-length proteins. HLA-class I antibody was used to block antigen-mediated TCR ligation.

sGFP induction in Vα3S1/Vβ13S1-TCR hybridoma cells was examined after 24 h coculture with antigen-presenting cells by UV-fluorescence microscopy and/or flow cytometry. As controls, Vα3S1/Vβ13S1-TCR hybridoma cells were cultured in anti-mouse CD3 antibody-coated (clone 17A2, 2 µg/ml in PBS, eBioscience) culture plates. pRSV-GFP or pcDNA-GFP served as transfection controls.

HLA-C*06:02-negative antigen-presenting cells or cell lines were transfected with pRSV-HLA-C*06:02 or pRSV-HLA-A*0201. Cell lines were transfected with plasmid-encoded peptides or full-length proteins. HLA-class I antibody was used to block antigen-mediated TCR ligation.

### 1.5 Identification of natural candidate peptide antigens based on mimotope sequences

Two different search strategies were used to identify human candidate peptide epitopes based on the conserved amino acid motifs of the mimotopes. We employed two tools from the "The European Molecular Biology Open Software Suite" (http://emboss.sourceforge.net/): "PROPHECY" (http://emboss.sourceforge.net/apps/release/6.6/emboss/apps/prophecy.html) was used to create a frequency matrix from sequences of positively tested antigenic peptides. With this matrix and the "PROFIT" tool (http://emboss.sourceforge.net/apps/release/6.6/emboss/apps/profit.html), the taxa-specific *Homo sapiens* [9606] UniProt database was scanned. The list of results was further refined with information on anchor amino acids of HLA-C*06:02 ligands (see Dionne et al., 2004; Falk et al., 1993) (Syfpeithi database at http://www.syfpeithi.de/). The matrix was constantly adjusted according to newly isolated mimotopes or tested candidate antigen peptides 1205Lu transcriptome data were used for a selective search in melanocytic proteins. The MOTIF Search web server (http://www.genome.jp/tools/motif/MOTIF2.html) was searched for human genes in the "KEGG Genes" dataset containing peptide motifs that were defined according to mimotope sequences and general HLA-C*06:02 anchor positions.

### 1.6 Dextramer staining

Fluorescent dextramers were prepared of HLA-C*06:02 and antigenic or control peptides. The control peptide has been originally described as an HLA-C*06:02-ligan by Falk et al⁶. PBMC from psoriasis patients were incubated with standard dilutions of the HLA-C*06:02-peptide dextramers and a phycoerythrin-labelled CD8 antibody and the frequency of double stained cells subsequently determined by flow cytometry on a FACScan flow cytometer. Data were analyzed by FlowJo software 887.

### 1.7 Peptide Stimulation of PBMC

PBMC were stimulated with synthetic peptides (10ng/ml) for 48 hrs. Intracellular cytokine staining (Fujii et al., 2011) employed antibodies against CD8, IL-17A and IFN-γ. Data were acquired by flow cytometry and analysed by Flow Jo software. INF-γ and IL-17A levels in culture supernatants were determined by ELISA.

### 1.8 Statistics

Kruskal-Wallis H-test was used for multiple comparisons and Bonferroni correction was applied. When *p*-value of Kruskal-Wallis H-test was significant, unpaired data of two groups were compared using Mann-Whitney U-test. Differences in the two groups were compared with Fisher's exact test. Two-tailed *p*-values <0.05 were considered significant.

The features disclosed in the foregoing description, in the claims and/or in the accompanying drawings may, both separately and in any combination thereof, be material for realizing the invention in diverse forms thereof.

### REFERENCES

Altman JD, Moss PA, Goulder PJ, et al. Phenotypic analysis of antigen-specific T lymphocytes. Science 1996; 274(5284): 94-96.
Arden, B., Clark, S.P., Kabelitz, D., and Mak, T.W. 1995. Human T-cell receptor variable gene segment families. Immunogenetics 42:455-500.
Birnbaum, M.E., Mendoza, J.L., Sethi, D.K., Dong, S., Glanville, J., Dobbins, J., Ozkan, E., Davis, M.M., Wucherpfennig, K.W., and Garcia, K.C. 2014. Deconstructing the peptide-MHC specificity of T cell recognition. Cell 157:1073-1087.
Boukamp, P., Petrussevska, R.T., Breitkreutz, D., Hornung, J., Markham, A., and Fusenig, N.E. 1988. Normal keratinization in a spontaneously immortalized aneuploid human keratinocyte cell line. J Cell Biol 106:761-771.
Chang, J.C., Smith, L.R., Froning, K.J., Kurland, H.H., Schwabe, B.J., Blumeyer, K.K., Karasek, M.A., Wilkinson, D.I., Farber, E.M., Carlo, D.J., et al. 1997. Persistence of T-cell clones in psoriatic lesions. Arch Dermatol 133:703-708.
Chiricozzi, A., and Krueger, J.G. 2013. IL-17 targeted therapies for psoriasis. Expert Opin Investig Drugs 22:993-1005.
Cole DK, Miles KM, Madura F, Holland CJ, Schauenburg AJ, Godkin AJ, Bulek AM, Fuller A, Akpovwa HJ, Pymm PG, Liddy N, Sami M, Li Y, Rizkallah PJ, Jakobsen BK, Sewell AK.T-cell receptor (TCR)-peptide specificity overrides affinity-enhancing TCR-major histocompatibility complex interactions. J Biol Chem. 2014;289(2):628-38.
Conrad, C., Boyman, O., Tonel, G., Tun-Kyi, A., Laggner, U., de Fougerolles, A., Kotelianski, V., Gardner, H., and Nestle, F.O. 2007. Alpha1beta1 integrin is crucial for accumulation of epidermal T cells and the development of psoriasis. Nat Med 13:836-842.
Dal Porto J, Johansen TE, Catipović B, et al. A soluble divalent class I major histocompatibility complex molecule inhibits alloreactive T cells at nanomolar concentrations. Proc Natl Acad Sci USA 1993; 90(14): 6671-6675.
Davidson, A., and Diamond, B. 2001. Autoimmune diseases. N Engl J Med 345:340-350.
Davis, M.M., and Bjorkman, P.J. 1988. T-cell antigen receptor genes and T-cell recognition. Nature 334:395-402.
Di Cesare, A., Di Meglio, P., and Nestle, F.O. 2009. The IL-23/Th17 axis in the immunopathogenesis of psoriasis. J Invest Dermatol 129:1339-1350.
Dionne, S.O., Lake, D.F., Grimes, W.J. & Smith, M.H 2004. Identification of HLA-Cw6.02 and HLA-Cw7.01 allele-specific binding motifs by screening synthetic peptide libraries. Immunogenetics 56, 391-398
Falk, K., Rotzschke, O., Grahovac, B., Schendel, D., Stevanovic, S., Gnau, V., Jung, G., Strominger, J.L., and Rammensee, H.G. 1993. Allele-specific peptide ligand motifs of HLA-C molecules. Proc Natl Acad Sci USA 90:12005-12009.
Fujii, H., Arakawa, A., Kitoh, A., Miyara, M., Kato, M., Kore-eda, S., Sakaguchi, S., Miyachi, Y., Tanioka, M., and Ono, M. (2011). Perturbations of both nonregulatory and regulatory FOXP3+ T cells in patients with malignant melanoma. Br J Dermatol 164, 1052-1060.
Griffiths, C.E., and Barker, J.N. 2007. Pathogenesis and clinical features of psoriasis. Lancet 370:263-271.
Hsu, M.Y., and Herlyn, M. (1996). Cultivation of normal human epidermal melanocytes. Methods in molecular medicine 2, 9-20.
Icen, M., Crowson, C.S., McEvoy, M.T., Dann, F.J., Gabriel, S.E., and Maradit Kremers, H. 2009. Trends in incidence of adult-onset psoriasis over three decades: a population-based study. J Am Acad Dermatol 60:394-401.
Kent, S.C., Chen, Y., Bregoli, L., Clemmings, S.M., Kenyon, N.S., Ricordi, C., Hering, B.J., and Hafler, D.A. 2005. Expanded T cells from pancreatic lymph nodes of type 1 diabetic subjects recognize an insulin epitope. Nature 435:224-228.
Kim, S.M., Bhonsle, L., Besgen, P., Nickel, J., Backes, A., Held, K., Vollmer, S., Dornmair, K., and Prinz, J.C. 2012. Analysis of the Paired TCR alpha- and beta-chains of Single Human T Cells. PLoS ONE 7:e37338.
Nair, R.P., Stuart, P.E., Nistor, I., Hiremagalore, R., Chia, N.V., Jenisch, S., Weichenthal, M., Abecasis, G.R., Lim, H.W., Christophers, E., et al. 2006. Sequence and haplotype analysis supports HLA-C as the psoriasis susceptibility 1 gene. Am J Hum Genet 78:827-851.
Neefjes, J., Jongsma, M.L., Paul, P., and Bakke, O. 2011. Towards a systems understanding of MHC class I and MHC class II antigen presentation. Nat Rev Immunol 11:823-836.
Parisi, R., Symmons, D.P., Griffiths, C.E., and Ashcroft, D.M. 2013. Global epidemiology of psoriasis: a systematic review of incidence and prevalence. J Invest Dermatol 133:377-385*.*
Scholler, J. et al. A recombinant human HLA-class I antigen linked to dextran elicits innate and adaptive immune responses. J Immunol. Methods 360, 1-9 (2010).
Seitz, S., Schneider, C.K., Malotka, J., Nong, X., Engel, A.G., Wekerle, H., Hohlfeld, R., and Dornmair, K. 2006. Reconstitution of paired T cell receptor alpha- and beta-chains from microdissected single cells of human inflammatory tissues. Proc Natl Acad Sci U S A 103:12057-12062.
Siewert, K., Malotka, J., Kawakami, N., Wekerle, H., Hohlfeld, R., and Dornmair, K. 2011. Unbiased identification of target antigens of CD8+ T cells with combinatorial libraries coding for short peptides. Nat. Medicine 18:824-828.
Tan MP, Gerry AB, Brewer JE, Melchiori L, Bridgeman JS, Bennett AD, Pumphrey NJ, Jakobsen BK, Price DA, Ladell K, Sewell AK. T cell receptor binding affinity governs the functional profile of cancer-specific CD8+ T cells. Clin Exp Immunol. 2015;180(2):255-70.
Uchanska-Ziegler, B., Loll, B., Fabian, H., Hee, C.S., Saenger, W., and Ziegler, A. 2012. HLA class I-associated diseases with a suspected autoimmune etiology: HLA-B27 subtypes as a model system. Eur J Cell Biol 91:274-286.
Wucherpfennig, K.W., Allen, P.M., Celada, F., Cohen, I.R., De Boer, R., Garcia, K.C., Goldstein, B., Greenspan, R., Hafler, D., Hodgkin, P., et al. 2007. Polyspecificity of T cell and B cell receptor recognition. Semin Immunol 19:216-224.

## Claims

1. A peptide comprising an amino acid sequence of the general formula I
**X1ₙ - Arg - X2 - X3 - Y1 - X4 - Y2 - Arg - Z** (I)
wherein
**X1** is selected from Phe, Arg, Gly, Met, Ala, Ser, Leu or Val;
**n** is 0 or 1.
**X2** is selected from any amino acid;
**X3** is selected from any amino acid;
**X4** is selected from Thr, Tyr, Val, Cys, Ser, Ala;
**Y1** is selected from Arg, Lys, Gln, or Asn;
**Y2** is selected from Arg, Leu or Ser; and
**Z** is selected from Leu, Met, Ile or Val.

2. The peptide of claim 1, wherein the peptide has a length of at least 8 or 9 amino acids, preferably about 8 to 150 amino acids or about 9 to 150 amino acids,
more preferably about 8 to 100 amino acids or about 9 to 100 amino acids,
more preferably about 8 to 50 or about 9 to 50 amino acids,
more preferably about 8 to 20 or about 9 to 20 amino acids,
even more preferably about 8 to 15 or about 9 to 15 amino acids, such as up to 12 or 13 amino acids;
and/or wherein the amino acids of the peptide are either L-amino acids or D-amino acids.

3. The peptide of claim 1 or 2, comprising further component(s), which is/are preferably selected from
N- and/or C-terminal modification(s),
such as comprising acetylation and/or amidation of the N- and/or C-terminus, label(s),
such as fluorescent dye(s), radioisotope(s) and/or contrast agent(s);
tag(s);
drug(s) or their respective prodrug(s);
carrier or depot(s) for drug(s), prodrug(s) or label(s);
immunogenic epitope(s);
cytotoxin(s),
such as saporin, gelonin or ricin;
radionuclides,
such as actinium-225, astatine-211, bismuth-213, iodide-131, optionally with a chelate group
and/or combinations thereof
preferably covalently attached, such as via an amino acid side chain, a linker, spacer and/or anchor group(s), e.g. a cleavable linker;
and/or wherein the peptide comprises or is an antigen causing a T cell triggered immune response, preferably a CD8⁺ T cell antigen.

4. The peptide of any one of claims 1 to 3, wherein
**X2** is selected from Ser, His, Cys, Tip, Asn, Ala, Tyr, Gln, or Phe;
such as
selected from Ser, His, Cys, Tip, Asn, Ala, or Tyr; or
selected from His, Gln, Ser, Ala or Phe;
and/or
**X3** is selected from Tyr, Arg, Trp, Ser, Ala, His, Phe, Val, Gly, or Cys;
such as
selected from Tyr, Arg, Trp, Ser, Ala, His, Phe, or Ala; or
selected from Val, Gly, Arg, Ser or Cys;
and/or
**X1,** if present, is selected from Phe, Arg, Gly, Met, Ala, Ser, Leu or Val;
such as
selected from Phe, Arg, Gly, or Met; or
selected from Ala, Arg, Ser or Leu;
and/or
**X4** is selected from Thr, Tyr, Val, Cys, Ser, Ala;
such as
selected from Thr, Tyr, Val, or Cys; or
selected from Ser, Ala, Cys or Thr;
and/or
**Y2** is selected from Arg, Leu or Ser; and
such as
selected from Arg, or Leu; or
selected from Arg, Leu or Ser.

5. The peptide of any one of claims 1 to 4, wherein
**Z** is Leu; or
**Y2** is Arg; or
**Y2** is Arg and **Z** is Leu, Met, Ile or Val; or
**Y2** is Arg and **Z** is Leu;
preferably comprising an amino acid sequence selected from the following formulas
**X1 - Arg - X2 - X3 - Y1 - X4 - Y2 - Arg - Leu** (Ia)
**X1 - Arg - X2 - X3 - Y1 - X4 - Arg - Arg - Z** (Ib)
**X1 - Arg - X2 - X3 - Y1 - X4 - Arg - Arg - Leu** (Ia)
wherein
**X1, X2, X3, X4** and **Y1** are as defined any of claims 1, 5 or 6;
**Y2** is selected from Arg, or Leu;
**Z** is selected from Leu, Met, Ile or Val;
and/or wherein the peptide preferably comprises or consists of an amino acid sequence selected from the group of SEQ ID NOs. 1 to 19.

6. A peptide- HLA-C monomer complex,
comprising
(a) at least one peptide of any one of claims 1 to 5,
(b) a HLA-C monomer binding to T-cell receptor(s) (TCR(s)).

7. A peptide- HLA-C multimer complex,
comprising a HLA-C multimer which comprises at least two HLA-C monomers as defined in claim 9 as (b) and which each comprises at least one peptide of any one of claims 1 to 5,
which is preferably a HLA-C*06:02 multimer, more preferably a HLA-C*06:02 tetramer or a HLA-C*06:02 dextramer,
preferably comprising 10 to 25 HLA-C monomers,
or preferably comprising 2 to 10 HLA-C monomers.

8. The complex of claim 6 or 7, wherein the HLA-C comprises further component(s), such as
label(s),
such as fluorescent dye(s), radioisotope(s) and/or contrast agent(s);
tag(s);
drug(s) or their respective prodrug(s);
carrier or depot(s) for drug(s), prodrug(s) or label(s);
immunogenic epitope(s);
cytotoxin(s),
such as saporin, gelonin or ricin;
radionuclides,
such as actinium-225, astatine-211, bismuth-213, iodide-131, optionally with a chelate group
coupling moiety/moieties
such as for obtaining HLA-C multimers,
such as dextrane, biotin, streptavidin, IgG-Fc region(s),
and/or combinations thereof
preferably covalently attached, such as via an amino acid side chain, a linker, spacer and/or anchor group(s), e.g. a cleavable linker.

9. The complex of any one of claims 6 to 8, wherein the at least one peptide (a) is altered for modifying the TCR binding affinity of the peptide or the peptide- HLA-C complex.

10. A pharmaceutical composition, comprising
(i) at least one peptide of any one of claims 1 to 5 and/or a HLA-C monomer as defined in claim 6 or 8; or
a peptide- HLA-C monomer complex or peptide- HLA-C multimer complex of any one of claims 6 to 9;
(ii) optionally, a pharmaceutically acceptable carrier and/or excipient.

11. The pharmaceutical composition of claim 10, comprising two, three, four or more peptides of any one of claims 1 to 5.

12. Use of a peptide of any one of claims 1 to 5, a monomer or multimer complex of any one of claims 6 to 9, a pharmaceutical composition of claim 10 or 11 or a protein comprising an amino acid sequence of SEQ ID NOs. 22 to 31 as biomarker for Psoriasis,
preferably comprising at least one of the following:
- monitoring disease activity;
- monitoring efficacy of treatment;
- determining disease risk; and/or
- determining the frequency of autoreactive T cells in samples of subjects having Psoriasis, preferably Psoriasis vulgaris with all types of manifestations, such as but not limited to chronic plaque psoriasis, acute guttate psoriasis, localized or generalized pustular psoriasis, psoriatic arthritis, psoriatic nail disease and others.

13. The peptide of any one of claims 1 to 5, the monomer or multimer complex of any one of claims 6 to 9, or the pharmaceutical composition of claim 10 or 11 for use in medicine.

14. The peptide of any one of claims 1 to 5, the monomer or multimer complex of any one of claims 6 to 9, or the pharmaceutical composition of claim 10 or 11 or a protein comprising an amino acid sequence of SEQ ID NOs. 22 to 31
for use in the diagnosis, prevention, monitoring and/or treatment of Psoriasis, preferably Psoriasis vulgaris.

15. The peptide or pharmaceutical composition or complex or protein for use according to claim 14, wherein the monitoring of Psoriasis, preferably Psoriasis vulgaris, comprises monitoring the treatment or therapy of Psoriasis, preferably Psoriasis vulgaris.

16. The peptide or pharmaceutical composition or complex or protein for use according to claim 14, comprising:
determining the frequency of autoreactive T cells in samples of subjects having Psoriasis, preferably Psoriasis vulgaris.

17. The peptide or pharmaceutical composition or protein for use according to claim 14, wherein the prevention and/or treatment of Psoriasis, preferably Psoriasis vulgaris, comprises
(a) inhibiting or blocking the interaction of TCR and HLA-C, and/or
(b) suppressing the expression of HLA-C.

18. The complex for use according to claim 14, wherein the prevention and/or treatment of Psoriasis, preferably Psoriasis vulgaris, comprises
creating immunotolerance against the autoantigens;
affecting, tolerating or blocking the activation of autoreactive T cells for therapeutic purposes;
eliminating the pathogenic CD8⁺ T cells; and/or
down-regulating the pathogenic immune response by specifically blocking HLA-C/T-cell-interactions.

19. A compound that is directed against HLA-C
for use in the prevention, and/or treatment of Psoriasis, preferably Psoriasis vulgaris,
wherein said compound is selected from
- an anti-HLA-C antibody or fragment thereof (such as Fab, Fv, scFv),
- a small molecule (inhibitor),
- a small interfering RNA;
wherein, preferably, the prevention and/or treatment of Psoriasis, preferably Psoriasis vulgaris, comprises
(a) inhibiting or blocking the interaction of TCR and HLA-C, and/or
(b) suppressing the expression of HLA-C.

20. A method for the prevention and/or treatment of Psoriasis, preferably Psoriasis vulgaris, comprising
(a) inhibiting or blocking the interaction of TCR and HLA-C, and/or
(b) suppressing the expression of HLA-C.

21. The compound for use according to claim 19 or the method of claim 20, wherein
(a) inhibiting or blocking the interaction of TCR and HLA-C comprises interfering with the contact between TCR and HLA-C
such as but not limited to antibodies (monoclonal antibodies, antibody fragments, e.g. Fab, Fv, scFv) or other molecules directed specifically against HLA-C
(b) suppressing the expression of HLA-C comprises
suppressing the expression of HLA-C on cell surfaces by means such as but not limited to small interfering RNAs or other small molecules that are designed to reduce HLA-C transcription, HLA-C translation or HLA-C transport to the cell surface.
